# EUROPEAN PATENT APPLICATION

(11) **EP 4 410 982 A1**
(43) Date of publication of application: **07.08.2024**
(21) Application number: 22873041.2
(22) Date of filing: 26.09.2022
(51) Int. Cl.: C12N 15/67, C07K 5/103, C07K 7/06, C07K 19/00, C12N 15/63, C12P 21/02, C12P 21/04, C40B 40/08, C40B 40/10

(54) **METHOD FOR PRODUCING POLYPEPTIDE, TAG, EXPRESSION VECTOR, METHOD FOR EVALUATING POLYPEPTIDE, METHOD FOR PRODUCING NUCLEIC ACID DISPLAY LIBRARY, AND SCREENING METHOD**

(30) Priority: 27.09.2021 JP 2021157186
(71) Applicant: FUJIFILM Corporation, Tokyo 106-8620 (JP)
(72) Inventor: MATSUI, Ken, Ashigarakami-gun, Kanagawa 258-8577 (JP); TOMINAGA, Jo, Ashigarakami-gun, Kanagawa 258-8577 (JP); KIHARA, Shiori, Ashigarakami-gun, Kanagawa 258-8577 (JP); HOSAKA, Takahiro, Nomi-shi, Ishikawa 923-1292 (JP); WATANABE, Takayoshi, Nomi-shi, Ishikawa 923-1292 (JP)
(74) Representative: Hoffmann Eitle
(86) International application number: PCT/JP2022/035787
(87) International publication number: WO 2023/048290

(57) **Abstract**

Provided is a production method of a polypeptide in which expressing a polypeptide as a tagged polypeptide is included, the method including expressing the tagged polypeptide from a nucleic acid containing a base sequence encoding a tag consisting of an amino acid sequence set forth in SEQ ID NO: 1, which is arranged immediately after a start codon, and a base sequence encoding a polypeptide that is arranged in-frame downstream of the base sequence encoding the tag,
SEQ ID NO: 1: Val-Lys-Lys-(Xaa)n. (Xaa)n is a chain of n pieces of any amino acids, where n is an integer of 1 to 8, and the amino acids constituting (Xaa)n may be one type of amino acid or two or more types of amino acids.

## Description

### BACKGROUND OF THE INVENTION

### 1. Field of the Invention

The present disclosure relates to a production method of a polypeptide, a tag, an expression vector, an evaluation method of a polypeptide, a production method of a nucleic acid display library, and a screening method.

### 2. Description of the Related Art

In a case of expressing a target protein by genetic engineering, there is a technique of attaching a relatively low molecular weight polypeptide, which is called a tag, to the terminal of the target protein. The tag is used to detect, isolate, immobilize, or the like a target protein.

It is being examined to use the tag to increase the expression level of a target protein.

WO2016/204198A discloses a tag (SEQ ID NO: 107) consisting of an amino acid sequence of SKIK as a tag that increases an expression level of a target protein and a method of adding the tag to the N-terminal of the target protein to express the target protein.

Protein Expression and Purification 2010; 74: 248-256 describes that an expression level of a protein is increased by incorporating Ala or Ser immediately after a start codon.

The optimization of the base sequence of the gene has been also examined in order to increase the expression level of the protein.

Science 2013; 342: 475-479 describes that the use of rare codons at the N-terminal of a protein increases the expression level thereof in Escherichia coli.

Protein Expression and Purification 2010; 70: 224-230 describes that regarding human peptide deformylase (hPDF), a reduction of the GC content of the hPDF gene and removal of rare codons increases an expression level in Escherichia coli.

### SUMMARY OF THE INVENTION

The embodiment according to the present disclosure relates to a production method of a polypeptide having high productivity, a tag and an expression vector capable of increasing an expression level of a polypeptide, an efficient evaluation method of a polypeptide, a production method of a nucleic acid display library in which an expression level is high and a variation of the expression level is small, and a screening method with a high reliability degree.

The specific methods for achieving the object include the following aspects.
<1> A production method of a polypeptide in which expressing a polypeptide as a tagged polypeptide is included, the production method comprising:
   expressing the tagged polypeptide from a nucleic acid containing
   a base sequence encoding a tag consisting of an amino acid sequence set forth in SEQ ID NO: 1, which is arranged immediately after a start codon, and
   a base sequence encoding the polypeptide that is arranged in-frame downstream of the base sequence encoding the tag.
<2> The production method of a polypeptide according to <1>, in which the tag is a tag consisting of an amino acid sequence set forth in SEQ ID NO: 2.
<3> The production method of a polypeptide according to <1>, in which the tag is a tag consisting of an amino acid sequence set forth in SEQ ID NO: 3.
<4> The production method of a polypeptide according to any one of <1> to <3>, in which the tagged polypeptide is expressed from the nucleic acid by a cell-free peptide synthesis system or Escherichia coli.
<5> The production method of a polypeptide according to any one of <1> to <4>, in which the polypeptide contains an unnatural amino acid.
<6> A tag consisting of an amino acid sequence set forth in SEQ ID NO: 1.
<7> A tag consisting of an amino acid sequence set forth in SEQ ID NO: 2.
<8> A tag consisting of an amino acid sequence set forth in SEQ ID NO: 3.
<9> The tag according to any one of <6> to <8>, in which the tag is used for expressing a polypeptide as a tagged polypeptide by a cell-free peptide synthesis system or Escherichia coli.
<10> The tag according to <9>, in which the polypeptide contains an unnatural amino acid.
<11> An expression vector comprising a base sequence encoding a tag consisting of an amino acid sequence set forth in SEQ ID NO: 1, which is arranged immediately after a start codon, and a base sequence encoding a polypeptide that is arranged in-frame downstream of the base sequence encoding the tag.
<12> The expression vector according to <11>, in which the tag is a tag consisting of an amino acid sequence set forth in SEQ ID NO: 2.
<13> The expression vector according to <11>, in which the tag is a tag consisting of an amino acid sequence set forth in SEQ ID NO: 3.
<14> The expression vector according to any one of <11> to <13>, in which the expression vector is designed to be expressed in a cell-free peptide synthesis system or Escherichia coli.
<15> An evaluation method of a polypeptide, comprising producing a polypeptide by the production method of a polypeptide according to any one of <1> to <5> and evaluating binding property of the polypeptide to a target substance.
<16> production method of a nucleic acid display library comprising producing a nucleic acid-polypeptide conjugate by expressing a tagged polypeptide from a nucleic acid containing a base sequence encoding a tag consisting of an amino acid sequence set forth in SEQ ID NO: 1, which is arranged immediately after a start codon, and a base sequence encoding a polypeptide that is arranged in-frame downstream of the base sequence encoding the tag.
<17> The production method of a nucleic acid display library according to <16>, in which the tag is a tag consisting of an amino acid sequence set forth in SEQ ID NO: 2.
<18> The production method of a nucleic acid display library according to <16>, in which the tag is a tag consisting of an amino acid sequence set forth in SEQ ID NO: 3.
<19> The production method of a nucleic acid display library according to any one of <16> to <18>, in which the production of the nucleic acid-polypeptide conjugate includes producing an mRNA-polypeptide conjugate and reverse-transcribing an mRNA of the mRNA-polypeptide conjugate to produce a cDNA-polypeptide conjugate.
<20> The production method of a nucleic acid display library according to any one of <16> to <19>, in which in the nucleic acid-polypeptide conjugate, the nucleic acid and the polypeptide are linked by a puromycin linker.
<21> The production method of a nucleic acid display library according to any one of <16> to <20>, in which the polypeptide contains an unnatural amino acid.
<22> The production method of a nucleic acid display library according to any one of <16> to <21>, in which the polypeptide is a polypeptide containing an amino acid having a first functional group and an amino acid having a second functional group that is covalently bonded to the first functional group, in which 2 to 28 amino acids are interposed between the amino acid having the first functional group and the amino acid having the second functional group, and
   the production method further includes allowing the polypeptide of the nucleic acid-polypeptide conjugate to cyclize by a covalent bond between the first functional group and the second functional group.
<23> A screening method comprising
   producing a nucleic acid display library by the production method of a nucleic acid display library according to any one of <16> to <22>, and
   selecting a nucleic acid-polypeptide conjugate having a target activity from the nucleic acid display library and identifying a base sequence of a nucleic acid of the selected nucleic acid-polypeptide conjugate.

According to the embodiment according to the present disclosure, a production method of a polypeptide having high productivity, a tag and an expression vector capable of increasing an expression level of a polypeptide, an efficient evaluation method of a polypeptide, a production method of a nucleic acid display library in which an expression level is high and a variation of the expression level is small, and a screening method with a high reliability degree are provided.

### BRIEF DESCRIPTION OF THE DRAWINGS

Fig. 1 is a graph showing a biosynthesis amount of a fusion protein for tags having SEQ ID NOs: 7 to 26, in which the tags are arranged in the order of the biosynthesis amounts of the fusion proteins.
Fig. 2 is a graph showing a biosynthesis amount of a fusion protein for the tags having SEQ ID NOs: 27, 28, 31, 37, 48, and 52.
Fig. 3 is a graph showing a binding performance of an integrin binding peptide to which a tag of SEQ ID NO: 27 is added.
Fig. 4 is a diagram showing structures of compounds introduced into SEQ ID NOs: 133 to 136.

### DESCRIPTION OF THE PREFERRED EMBODIMENTS

Hereinafter, embodiments according to the present disclosure will be described. These descriptions and Examples are only illustrative of one embodiment and do not limit the ranges of the embodiments of the present disclosure. The action mechanism mentioned in the present disclosure includes estimation, and the accuracy thereof does not limit the ranges of the embodiments of the present disclosure.

In the present disclosure, a numerical range expressed using "to" indicates a range including numerical values before and after "to" as a minimum value and a maximum value.

In the numerical ranges described stepwise in the present disclosure, the upper limit value or the lower limit value described in one numerical range may be replaced with the upper limit value or the lower limit value of the numerical range described stepwise in other stages. In addition, in a numerical range described in the present disclosure, an upper limit value or a lower limit value described in the numerical range may be replaced with a value described in an example.

In the present disclosure, each component may contain a plurality of types of substances corresponding thereto. In the present disclosure, upon referring to an amount of each component in a composition, the amount means a total amount of a plurality of substances present in the composition unless otherwise specified, in a case where a plurality of substances corresponding to each component are present in the composition.

In the present disclosure, a polypeptide refers to a molecule in which amino acids are linked by peptide bonds. The number of amino acid residues of the polypeptide is not limited, and the polypeptide is a term including a protein. It is desirable that the polypeptide in the present disclosure has 6 or more residues of amino acid residues. The polypeptide includes a polypeptide in which an amino acid is post-translationally modified. Examples of the post-translation modification of an amino acid include phosphorylation, methylation, acetylation, and the like.

In the present disclosure, a nucleic acid refers to a molecule carrying information for synthesizing a polypeptide. The nucleic acid is a term including any nucleic acid (for example, DNA, RNA, an analog thereof, a natural product, or an artificial product) and a nucleic acid in which a low-molecular-weight compound, a group, a molecule other than a nucleic acid, a structure, or the like is linked to any nucleic acid. The nucleic acid may be a single-stranded nucleic acid or a double-stranded nucleic acid.

In the present disclosure, in the notation of an amino acid, the three-letter notation and the one-letter notation established by IUPAC-IUBMB joint commission on Biochemical Nomenclature (IUPAC-IUBMB JCBN) are used.

Unless otherwise specified, the amino acid referred to in the present disclosure is an L-amino acid.

### <Production method of polypeptide, and tag and expression vector>

The present disclosure provides a production method of a polypeptide having high productivity. The production method of a polypeptide according to an embodiment of the present disclosure includes expressing the polypeptide as a tagged polypeptide.

The present disclosure provides a tag and an expression vector that increase an expression level of a polypeptide for a production method of the polypeptide having high productivity. The tags provided by the embodiment according to the present disclosure are collectively referred to as VKKX tag.

A production method of a polypeptide according to the present disclosure includes expressing a tagged polypeptide from a nucleic acid that has a base sequence encoding a VKKX tag arranged immediately after a start codon and a base sequence encoding a polypeptide arranged in-frame downstream of the base sequence encoding the VKKX tag. The base sequence encoding the VKKX tag and the base sequence encoding the polypeptide are arranged in frame in one molecule of nucleic acid. That is, the VKKX tag and the polypeptide enter a reading frame with a start codon as a starting point immediately before the base sequence encoding the VKKX tag. The base sequence encoding the VKKX tag and the base sequence encoding the polypeptide may be directly linked, or may be linked through another base sequence.

It is presumed that the VKKX tag arranged immediately after the start codon increases the translation efficiency of the base sequence after the start codon. Therefore, the production method of a polypeptide according to the present disclosure is a production method of a polypeptide with high productivity.

The VKKX tag is a tag consisting of an amino acid sequence set forth in SEQ ID NO: 1. The VKKX tag is preferably a tag consisting of the amino acid sequence set forth in SEQ ID NO: 2, more preferably a tag consisting of the amino acid sequence set forth in SEQ ID NO: 3, and still more preferably a tag consisting of the amino acid sequence set forth in SEQ ID NO: 4.

SEQ ID NO: 1: Val-Lys-Lys-(Xaa)n (Xaa)n is a chain of n pieces of any amino acids, where n is an integer of 1 to 8, and the amino acids constituting (Xaa)n may be one type of amino acid or two or more types of amino acids.

In SEQ ID NO: 1, the amino acid constituting (Xaa)n is preferably n pieces of amino acids selected from the group consisting of Ile, Lys, Arg, His, Ser, Thr, Asp, Cys, Asn, Tyr, Gln, Trp, and Phe, and more preferably n pieces of amino acids selected from the group consisting of Ile, Lys, Arg, His, Ser, Thr, and Asp.

In SEQ ID NO: 1, n is preferably an integer of 1 to 7, and more preferably an integer of 2 to 7.

SEQ ID NO: 2: Val-Lys-Lys-Xaa-(Xaa)m Xaa at the fourth position from the N-terminal is one type of amino acid selected from the group consisting of Ile, Lys, Arg, His, Ser, and Thr, (Xaa)m is a chain of m pieces of any amino acids, where m is an integer of 0 to 7, and the amino acids constituting (Xaa)m may be one type of amino acid or two or more types of amino acids.

In SEQ ID NO: 2, Xaa at the fourth position from the N-terminal is preferably one type of amino acid selected from the group consisting of Ile, Lys, and Thr, and more preferably Thr.

In SEQ ID NO: 2, the amino acid constituting (Xaa)m is preferably m pieces of amino acids selected from the group consisting of Ile, Lys, Arg, His, Ser, Thr, Asp, Cys, Asn, Tyr, Gln, Trp, and Phe, and more preferably m pieces of amino acids selected from the group consisting of Ile, Lys, Arg, His, Ser, Thr, and Asp.

In SEQ ID NO: 2, m is preferably an integer of 0 to 6, and more preferably an integer of 1 to 6.

SEQ ID NO: 3: Val-Lys-Lys-Xaa-(Xaa)k Xaa at the fourth position from the N-terminal is one type of amino acid selected from the group consisting of Ile, Lys, and Thr, (Xaa)k is a chain of k pieces of amino acids selected from the group consisting of Lys, Thr, and Asp, where k is an integer of 0 to 6, and the amino acids constituting (Xaa)k may be one type of amino acid or two or more types of amino acids.

In SEQ ID NO: 3, Xaa at the fourth position from the N-terminal is preferably Thr.

In SEQ ID NO: 3, k is preferably an integer of 1 to 6.

SEQ ID NO: 4: Val-Lys-Lys-Thr-Lys-Thr-(Xaa)j (Xaa)j is a chain of j pieces of amino acids selected from the group consisting of Lys, Thr, and Asp, where j is an integer of 0 to 4, and the amino acids constituting (Xaa)j may be one type of amino acid or two or more types of amino acids.

Specific examples of the VKKX tag include a tag consisting of amino acid sequence of any one of SEQ ID NOs: 7 to 52.

The base sequence encoding the VKKX tag is not limited. For example, the base sequence of the VKKX tag can be designed according to a codon table of a biosynthesis system (for example, a cell-free peptide synthesis system, Escherichia coli) that is used for the expression of the tagged polypeptide.

The polypeptide which is a target product of the production method of a polypeptide according to the present disclosure is not limited in a three-dimensional structure, an amino acid sequence, the number of amino acid residues, the type of amino acid, and a base sequence encoding the polypeptide. The amino acid includes a natural amino acid, an unnatural amino acid, a modified amino acid, and a derivative thereof.

In the present disclosure, the natural amino acid refers to an amino acid that constitutes a general protein, and examples thereof include alanine (Ala, A), arginine (Arg, R), asparagine (Asn, N), aspartic acid (Asp, D), and cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V). The natural amino acid may be a natural product or an artificial product.

In the present disclosure, the unnatural amino acid refers to an amino acid other than the twenty types of amino acids described above, and includes a natural product or an artificial product.

An example of the unnatural amino acid is an amino acid having a chloroacetyl group (for example, a chloroacetylate amino acid such as a chloroacetyl lysine, a chloroacetyl diaminobutyric acid, and the like).

An example of the unnatural amino acid is an N-methylamino acid (for example, N-methylalanine and N-methylphenylalanine).

An example of the modified amino acid is a labeled amino acid which is an amino acid bonded to a labeling compound. The labeling compound is a substance that can be detected by a biochemical, chemical, immunochemical, or electromagnetic detection method. Examples of the labeling compound include a coloring agent compound, a fluorescent substance, a chemiluminescent substance, a bioluminescent substance, an enzyme substrate, a coenzyme, an antigenic substance, a substance that binds to a specific protein, and a magnetic substance. Examples of the labeled amino acid include, in a case of being classified by function, a fluorescent-labeled amino acid, a radioactive isotope-labeled amino acid, a photoresponsive amino acid, a photoswitch amino acid, and a fluorescent probe amino acid.

In the labeled amino acid, the amino acid and the labeling compound may be directly bonded to each other, or the amino acid and the labeling compound may be bonded through a spacer. Examples of the spacer include polyolefins such as polyethylene and polypropylene; polyethers such as polyoxyethylene, polyethylene glycol, and polyvinyl alcohol; polystyrene, polyvinyl chloride, polyester, polyamide, polyimide, polyurethane, polycarbonate, and the like.

Examples of the derivative of the natural amino acid, the unnatural amino acid, or the modified amino acid include hydroxy acids, mercapto acids, and carboxylic acids.

Examples of the embodiment of the polypeptide include a polypeptide containing an unnatural amino acid. Specific examples thereof include a polypeptide containing an N-methylamino acid (for example, N-methylalanine or N-methylphenylalanine). The number of amino acid residues of the polypeptide containing an unnatural amino acid is, for example, 3 to 20.

Examples of the embodiment of the polypeptide include a cyclic polypeptide containing an unnatural amino acid. Specific examples thereof include a polypeptide which contains an amino acid having a thiol group (for example, cysteine), an amino acid having a chloroacetyl group (for example, chloroacetyl diaminobutyric acid and chloroacetyl lysine), an N-methyl amino acid (for example, N-methylalanine, N-methylphenylalanine), in one molecule. As this polypeptide, the thiol group and the chloroacetyl group react with each other to be cyclized, resulting in a cyclic polypeptide. The number of amino acid residues of the cyclic polypeptide containing an unnatural amino acid is, for example, 3 to 20.

The VKKX tag and the polypeptide may be directly linked, or may be linked through another amino acid sequence. Examples of the other amino acid sequences include a protease recognition sequence (for example, a sortase recognition sequence, an HRV3C recognition sequence, and a TEV protease recognition sequence), a spacer sequence (for example, at least one amino acid selected from the group consisting of glycine and serine), a tag other than the VKKX tag (for example, a His tag, an HA tag, a FLAG tag, a Myc tag, and a HiBiT tag).

The protease recognition sequence enables the VKKX tag and the polypeptide to be separated from each other. The spacer sequence improves the flexibility of the tagged polypeptide. The other tags facilitate the purification or detection of the tagged polypeptide. The other tags may be on the C-terminal side of the polypeptide, not between the VKKX tag and the polypeptide.

Instead of a functional sequence such as these amino acid sequences, an amino acid sequence having no specific function may be interposed between the VKKX tag and the polypeptide, or may be added to the C-terminal side of the polypeptide.

The expression vector according to the present disclosure contains a base sequence encoding a VKKX tag arranged immediately after a start codon, and a base sequence encoding a polypeptide arranged in-frame downstream of the base sequence encoding the VKKX tag. The VKKX tag is a tag consisting of an amino acid sequence set forth in SEQ ID NO: 1. The VKKX tag is preferably a tag consisting of the amino acid sequence set forth in SEQ ID NO: 2, more preferably a tag consisting of the amino acid sequence set forth in SEQ ID NO: 3, and still more preferably a tag consisting of the amino acid sequence set forth in SEQ ID NO: 4.

The expression vector according to the present disclosure may have a base sequence required for synthesizing a polypeptide by a biosynthesis system. The base sequence required for polypeptide synthesis may include not only a start codon, a base sequence encoding a VKKX tag, and a base sequence encoding a polypeptide, but also for example, a promoter sequence and/or a ribosome binding sequence. In a case where another amino acid sequence is inserted between the VKKX tag and the polypeptide or on the C-terminal side of the polypeptide, the expression vector may also have a base sequence encoding another amino acid sequence.

A promoter sequence, a ribosome binding sequence, a start codon, or the like may be arranged in the expression vector according to the requirement of a biosynthesis system (for example, a cell-free peptide synthesis system, Escherichia coli) that expresses the expression vector.

The expression vector according to the present disclosure may be produced by linking a DNA fragment containing a promoter sequence, a ribosome binding sequence, a start codon, a base sequence encoding a VKKX tag, a base sequence encoding a polypeptide, and the like by a method such as overlap extension PCR.

Examples of the vector that is used for constructing the expression vector according to the present disclosure include a plasmid DNA, a cosmid DNA, a phage DNA, an animal virus vector, an insect virus vector, and the like. The expression vector may be linear or cyclic.

An example of an embodiment of the production method of a polypeptide according to the present disclosure is realized by culturing a transformant including an expression vector and collecting a tagged polypeptide or polypeptide from the culture product. The transformant may be produced by transforming a host with the expression vector according to the present disclosure. A host used for producing the transformant may be any of Escherichia coli, yeast, a fungus cell, an insect cell, an animal cell, or a plant cell.

An example of an embodiment of the production method of a polypeptide according to the present disclosure is realized by a cell-free peptide synthesis system. The cell-free peptide synthesis system is a reaction system for polypeptide synthesis, and is, without using cells themselves, (1) a system configured to perform translation of a nucleic acid, or (2) a system configured to perform transcription and translation of a nucleic acid. The cell-free peptide synthesis system is composed of a template nucleic acid, a ribosome, a factor and an enzyme for transcription and/or translation, enzymes that are necessary for the constitution of the system, various substrates, an energy source, a buffer solution, and salts. Examples of the factor and enzyme for transcription and/or translation include a substance derived from a prokaryotic cell such as Escherichia coli; and a substance derived from a eukaryotic cell such as wheat germ, an animal cell, or an insect cell.

In the cell-free peptide synthesis system, the template nucleic acid may be DNA or RNA. The template nucleic acid may be a single-stranded nucleic acid or a double-stranded nucleic acid. The template nucleic acid may be a linear nucleic acid or a circular nucleic acid. The template nucleic acid may be a nucleic acid in which a base sequence required for polypeptide synthesis is incorporated into a vector (a plasmid vector, a cosmid vector, or the like).

The template nucleic acid has a base sequence required to synthesize a polypeptide by a cell-free peptide synthesis system. The base sequence required for polypeptide synthesis is not only a coding region but also, for example, a promoter sequence and a ribosome binding sequence. The base sequence of the template nucleic acid may include a stop codon or may not include a stop codon. The stop codon means a codon that has no pairing with tRNA. In a case where the base sequence of the template nucleic acid does not contain a stop codon, an mRNA-ribosome-polypeptide conjugate can be formed.
(1) A cell-free peptide synthesis system that performs translation of a nucleic acid (for example, RNA) contains a ribosome, a translation initiation factor, a translation elongation factor, a translation termination factor, an aminoacyl-tRNA synthase, and a tRNA aminoacylated by an aminoacyl-tRNA synthase, and in a case of a system derived from Escherichia coli, further contains methionyl tRNA transformylase.
(2) A cell-free peptide synthesis system that performs transcription and translation of a nucleic acid (for example, DNA) contains, in addition to the constituent substances of (1), an RNA polymerase (for example, T7 RNA polymerase) and a nucleoside triphosphate that is a substrate of RNA polymerase.

In both (1) and (2), the mRNA-ribosome-polypeptide conjugate can be formed by removing the translation termination factor from the constituent substances.

Examples of the enzymes other than the factor and enzyme for transcription and/or translation include enzymes for regeneration of energy such as creatine kinase, myokinase, and nucleoside diphosphate kinase (NDPK); enzymes for decomposition of inorganic pyrophosphate generated by transcription and/or translation of inorganic pyrophosphatase or the like; and the like.

Examples of various substrates include a natural amino acid and/or an unnatural amino acid, and a nucleotide triphosphate, a creatine phosphate, a formylfolic acid as an energy source. Examples of the nucleotide triphosphate include ATP, GTP, CTP, and UTP. ATP and GTP are used in (1), and ATP, GTP, CTP, and UTP are used in (2).

As the buffer solution, a potassium phosphate buffer solution (pH 7.3) is commonly used. As the salts, potassium glutamate, ammonium chloride, magnesium acetate, calcium chloride, putrescine, spermidine, dithiothreitol (DTT), or the like is commonly used.

In the production method of a polypeptide according to the present disclosure, any known cell-free peptide synthesis system can be adopted. Examples of the commercially available product of the cell-free peptide synthesis system include PUREfrex (GeneFrontier, "PUREfrex" is a registered trademark), PURExpress In Vitro Protein Synthesis Kit (New England BioLabs), Human Cell-Free Protein Expression System (Takara Bio), Rapid Translation System (Roche), Expressway Cell-Free Expression System (Invitrogen).

The production method of a polypeptide according to the present disclosure may further include purifying a tagged polypeptide, concentrating the tagged polypeptide, cleaving a VKKX tag of the tagged polypeptide, modifying a target polypeptide, and the like.

### <Evaluation method for polypeptide>

An evaluation method of a polypeptide according to the present disclosure includes producing a polypeptide by the production method of a polypeptide according to the present disclosure and evaluating binding property of the polypeptide to a target substance. In the evaluation step, the polypeptide may be a tagged polypeptide to which a VKKX tag is added or a polypeptide in which the VKKX tag is removed from the tagged polypeptide.

A surface plasmon resonance method is known as a method of evaluating the binding ability of a polypeptide to a target substance. However, in recent years, a method of acquiring a polypeptide using a cell-free peptide synthesis system and evaluating a binding ability by ELISA has been reported (Journal of Synthetic Organic Chemistry, Japan, 2017, vol. 75, No. 11, 1171-1178). However, in the cell-free peptide synthesis system, the amino acid sequence of the polypeptide may cause a bias in the expression level, and thus it is difficult to evaluate the binding of the polypeptide having a low expression level. According to the evaluation method of a polypeptide according to the present disclosure, it is possible to acquire a large expression level of the polypeptide and a small bias in the expression level among a plurality of types of polypeptides. Therefore, the binding ability and the like of more types of the polypeptide can be evaluated.

The target substance is a term including any chemical substance exhibiting physiological activity, and is a term including a compound, a group, a molecule, a protein, a nucleic acid, a lipid, a saccharide, a complex of these, and the like. The target substance is, for example, a metal ion, a lipid molecular aggregate, a peptide, a receptor, a transcription factor, an enzyme, a coenzyme, a regulating factor, an antibody, an antigen, DNA, RNA, a membrane vesicle, an extracellular vesicle, a cellular organ, a cell, a fragment thereof, a complex thereof, and a modification group thereof.

An example of an embodiment of the evaluation method of a polypeptide according to the present disclosure includes bringing the polypeptide into contact with a target substance (for example, a target protein) and performing incubation. For example, the polypeptide is brought into contact with the target substance in a buffer solution, and incubated with adjusted pH and temperature of the buffer solution. In this case, a target substance may be immobilized on a solid phase carrier, and then a polypeptide may be brought into contact with the immobilized target substance. Alternatively, the polypeptide may be immobilized on a solid phase carrier, and the target substance may be brought into contact with the immobilized polypeptide. The solid phase carrier is not limited as long as it is a carrier on which a target substance or a polypeptide can be immobilized, and examples thereof include a microtiter plate, a substrate, a bead, a magnetic bead, a nitrocellulose membrane, a nylon membrane, a PVDF membrane, and the like. The target substance or the polypeptide is immobilized on these solid phase carriers by a known technique.

After the contact described above, the polypeptide bound to the target substance (for example, the target protein) is quantified. For the quantification of the polypeptide, any known protein quantification technique can be adopted. For example, the polypeptide is quantified by gel electrophoresis, absorption spectrophotometry, fluorescence, enzyme-linked immunosorbent assay (ELISA), chromatography, a surface plasmon resonance method, or the like.

In an example of the embodiment of the evaluation method of a polypeptide according to the present disclosure, the polypeptide bound to the target substance is quantified by adding a tag other than the VKKX tag (for example, a His tag, an HA tag, a FLAG tag, a Myc tag, or a HiBiT tag) to the polypeptide to be evaluated, expressing the polypeptide, and using another function of tag (for example, luminescence).

### <Production method of nucleic acid display library>

The nucleic acid display library has a nucleic acid-polypeptide conjugate as a constitutional unit, and indicates a collection of a plurality of nucleic acid-polypeptide conjugates. The number of clones and the number of copies of the nucleic acid-polypeptide conjugate constituting the nucleic acid display library are not limited.

Examples of the embodiment of the nucleic acid-polypeptide conjugate include an mRNA-polypeptide conjugate or a cDNA-polypeptide conjugate. The cDNA-polypeptide conjugate is a reverse transcription product of the mRNA-polypeptide conjugate.

In the nucleic acid-polypeptide conjugate, the nucleic acid and the polypeptide are linked, for example, through a puromycin linker or a ribosome.

The polypeptide of the nucleic acid-polypeptide conjugate is not limited in a three-dimensional structure, an amino acid sequence, the number of amino acid residues, the type of amino acid, and a base sequence encoding the polypeptide. The amino acid includes a natural amino acid, an unnatural amino acid, a modified amino acid, and a derivative thereof.

In the nucleic acid display library produced by the production method of a nucleic acid display library according to the present disclosure, the polypeptide of the nucleic acid-polypeptide conjugate is expressed as a tagged polypeptide to which a VKKX tag is added.

A production method of a nucleic acid display library according to the present disclosure includes expressing a tagged polypeptide from a nucleic acid to produce a nucleic acid-polypeptide conjugate. The nucleic acid contains a base sequence encoding a VKKX tag arranged immediately after a start codon, and a base sequence encoding a polypeptide arranged in-frame downstream of the base sequence encoding the VKKX tag. The VKKX tag is a tag consisting of an amino acid sequence set forth in SEQ ID NO: 1. The VKKX tag is preferably a tag consisting of the amino acid sequence set forth in SEQ ID NO: 2, more preferably a tag consisting of the amino acid sequence set forth in SEQ ID NO: 3, and still more preferably a tag consisting of the amino acid sequence set forth in SEQ ID NO: 4.

In the production method of a nucleic acid display library according to the present disclosure, since a base sequence encoding a VKKX tag is arranged immediately after the start codon of the template nucleic acid, a nucleic acid display library having a large expression level and a small bias in the expression level can be produced.

As an example of the embodiment of the production method of a nucleic acid display library according to the present disclosure, the method includes producing an mRNA-polypeptide conjugate, and producing a cDNA-polypeptide conjugate by reverse transcription of mRNA of the mRNA-polypeptide conjugate. According to the examples of the present embodiment, since the nucleic acid of the nucleic acid-polypeptide conjugate is DNA, a chemically more stable nucleic acid display library can be obtained.

An example of an embodiment of the production method of a nucleic acid display library according to the present disclosure is carried out by a cell-free peptide synthesis system. The cell-free peptide synthesis system may be any of (1) a system configured to perform translation of a nucleic acid or (2) a system configured to perform transcription and translation of a nucleic acid. Details of the cell-free peptide synthesis system are as described above. In the production method of a nucleic acid display library according to the present disclosure, any known cell-free peptide synthesis system and any known nucleic acid display library production technique can be adopted.

In the production method of a nucleic acid display library according to the present disclosure, linking between a nucleic acid to be translated (usually mRNA) and a translation product may be any one of linking through a puromycin linker, linking through a ribosome, or the like. From the viewpoint that the translation product easily forms an appropriate higher-order structure and from the viewpoint that the function of the translation product is easily evaluated, the linking between the nucleic acid to be translated and the translation product is preferably a linking through a puromycin linker.

Accordingly, the nucleic acid to be translated in the cell-free peptide synthesis system is preferably a nucleic acid in which a puromycin linker is added to the 3' terminal. From the viewpoint of suppressing the dissociation of the nucleic acid and puromycin, the linker for adding puromycin to a nucleic acid is preferably a 2'-O-methylated nucleic acid linker or a nucleic acid linker having an ultraviolet crosslinking compound at the 5' terminal.

In the cell-free peptide synthesis system, the template nucleic acid may be DNA or RNA. The template nucleic acid is, for example, a population of double-stranded DNA fragments produced by performing overlap extension PCR using a random primer set including a random sequence. The random sequence is, for example, a repeating sequence [NNK]m of triplet (here, m is a positive integer, N's each independently represent A, T, G, or C, and K each independently represents T or G). By setting the number of repetitions of the triplet [NNK] to any number, a peptide having any length can be produced. From the viewpoint of suppressing the appearance of stop codon, the random sequence is preferably a trimer oligonucleotide in which one type of codon is assigned to one type of amino acid.

The template nucleic acid may include a base sequence encoding another amino acid sequence other than the VKKX tag and the target polypeptide. Examples of another amino acid sequence include a protease recognition sequence, a spacer sequence, and another tag other than the VKKX tag. The base sequence encoding another amino acid sequence is arranged in-frame between the base sequence encoding a VKKX tag and the base sequence encoding a target polypeptide or downstream of the base sequence encoding the target polypeptide.

From the viewpoint of increasing the flexibility of the polypeptide, a base sequence encoding a spacer is preferably present on the 3' terminus side of the template nucleic acid. The spacer is, for example, at least one amino acid selected from glycine or serine.

In an example of an embodiment of the production method of a nucleic acid display library according to the present disclosure, the polypeptide to be expressed is a polypeptide containing an unnatural amino acid. The unnatural amino acid includes an unnatural amino acid; a modified amino acid of a natural amino acid or an unnatural amino acid; or a derivative of a natural amino acid, an unnatural amino acid, or a modified amino acid. Details of these amino acids are as described above.

In an example of an embodiment of a production method of a nucleic acid display library according to the present disclosure, the polypeptide to be expressed is a polypeptide containing an amino acid having a first functional group and an amino acid having a second functional group that is covalently bonded to the first functional group, in which 2 to 28 amino acids are interposed between the amino acid having the first functional group and the amino acid having the second functional group. The polypeptide to be expressed may be a polypeptide in which 6 to 16 amino acids are interposed between the amino acid having the first functional group and the amino acid having the second functional group. As this polypeptide, the first functional group and the second functional group can react with each other to be cyclized, and can be formed into a cyclic polypeptide. The number of amino acid residues of the entire cyclic polypeptide is, for example, 4 to 32.

In a case where the polypeptide to be expressed is in the above-described form, the production method of the nucleic acid display library according to the present disclosure includes allowing the polypeptide of the nucleic acid-polypeptide conjugate to cyclize by a covalent bond between the first functional group and the second functional group. The cyclization may be formed in a mRNA-polypeptide conjugate or may be formed in a cDNA-polypeptide conjugate.

The first functional group and the second functional group may be the same type of functional group or different types of functional groups as long as the first functional group and the second functional group are covalently bonded to each other. Examples of the combination of the first functional group and the second functional group include a combination of a thiol group and a chloroacetyl group, a combination of a thiol group and a thiol group, and a combination of a carboxy group in a side chain and an amino group in a side chain.

Examples of the amino acid having a thiol group include cysteine.

Examples of the amino acid having a chloroacetyl group include chloroacetyl diaminobutyric acid and chloroacetyl lysine.

Examples of the amino acid having a carboxy group in the side chain include aspartic acid and glutamic acid.

Examples of the amino acid having an amino group in the side chain include lysine, asparagine, and glutamine.

### <Screening method>

A screening method according to the present disclosure includes producing a nucleic acid display library by the production method of a nucleic acid display library according to the present disclosure, and selecting a nucleic acid-polypeptide conjugate having a target activity from the nucleic acid display library and identifying a base sequence of a nucleic acid of the selected nucleic acid-polypeptide conjugate.

The "target activity" is, for example, binding to a target substance. The target substance is a term including any chemical substance exhibiting physiological activity, and is a term including a compound, a group, a molecule, a protein, a nucleic acid, a lipid, a saccharide, a complex of these, and the like. The target substance is, for example, a metal ion, a lipid molecular aggregate, a peptide, a receptor, a transcription factor, an enzyme, a coenzyme, a regulating factor, an antibody, an antigen, DNA, RNA, a membrane vesicle, an extracellular vesicle, a cellular organ, a cell, a fragment thereof, a complex thereof, and a modification group thereof.

An example of the embodiment of the screening method according to the present disclosure includes bringing a nucleic acid display library into contact with a target substance and performing incubation. For example, the nucleic acid display library is brought into contact with the target substance in a buffer solution, and incubated with adjusted pH and temperature of the buffer solution. In this case, a target substance may be immobilized on a solid phase carrier, and then a nucleic acid display library may be brought into contact with the immobilized target substance. The solid phase carrier is not limited as long as it is a carrier on which a target substance can be immobilized, and examples thereof include a microtiter plate, a substrate, a bead, a magnetic bead, a nitrocellulose membrane, a nylon membrane, a PVDF membrane, and the like. The target substance is immobilized on these solid phase carriers by a known technique.

After the contact described above, the nucleic acid-polypeptide conjugate bound to a target substance is extracted, and the base sequence of the nucleic acid of the extracted nucleic acid-polypeptide conjugate is identified. Identification of the base sequence can be carried out using a nucleic acid amplification system and a sequencer.

The nucleic acid amplification system refers to a system that amplifies a nucleic acid using a nucleic acid as a template. The nucleic acid amplification reaction of the nucleic acid amplification system can be any one of polymerase chain reaction (PCR), ligase chain reaction (LCR), transcription mediated amplification (TMA), nucleic acid sequence-based amplification (NASBA), or the like.

In the present disclosure, the sequencer is a term including a first generation sequencer (a capillary sequencer), a second generation sequencer (a next generation sequencer), a third generation sequencer, a fourth generation sequencer, and a sequencer to be developed in the future. The sequencer may be a capillary sequencer, may be a next generation sequencer, or may be another sequencer. The sequencer is preferably a next generation sequencer from the viewpoints of the speed of analysis, the large number of specimens that can be processed at one time, and the like. The next generation sequencer (NGS) refers to a sequencer that is classified by being contrasted with a capillary sequencer (called a first generation sequencer) using the Sanger method. At present, the most popular next generation sequencer is a sequencer of which the principle is to capture fluorescence or luminescence linked to a complementary strand synthesis by DNA polymerase or a complementary strand binding by DNA ligase and determine the base sequence. Specific examples thereof include MiSeq (Illumina, Inc., MiSeq is a registered trademark), HiSeq 2000 (Illumina, Inc., HiSeq is a registered trademark), and Roche 454 (Roche, Ltd.).

The screening method according to the present disclosure is a screening method with a high reliability degree since a nucleic acid display library having a large expression level and a small bias in the expression level is used as a population.

### Examples

The tag and the like according to the present disclosure will be described in more detail below with reference to specific examples. The materials, treatment procedures, and the like shown in the specific examples below can be changed as appropriate without departing from the gist of the present disclosure. The scope of the tag and the like according to the present disclosure should not be construed as being limited by the following specific examples.

In the following description, unless otherwise specified, the synthesis, treatment, manufacture, and the like were performed at room temperature (25°C ± 3°C). The percentage related to the substance concentration is based on mass.

### <Example 1: Examination of amino acid sequence of tag>

In the cell-free peptide synthesis system derived from Escherichia coli, the effect of the VKKX tag on the biosynthesis amount of the polypeptide was examined. As a polypeptide to be biosynthesized, an integrin binding peptide containing cysteine and chloroacetyl lysine was selected. In this polypeptide, a thiol group of cysteine and a chloroacetyl group of chloroacetyl lysine spontaneously form a thioether bond to become a cyclic polypeptide.

A fusion protein in which an integrin binding peptide, a Myc tag, and a HiBiT tag were linked in this order was designed. As a spacer, Gly-Gly-Ser was inserted between the integrin binding peptide and the Myc tag and between the Myc tag and the HiBiT tag. The amino acid sequence of this fusion protein is SEQ ID NO: 5, and the base sequence encoding this fusion protein is SEQ ID NO: 6. In SEQ ID NO: 5, the integrin binding peptide ranges to the amino acid at the twelfth position (that is, alanine) from an N-terminal, and the amino acid at eleventh position from the N-terminal is chloroacetyl lysine. The triplet TAG at the positions 31 to 33 from the 5' terminal of the base sequence set forth in SEQ ID NO: 6 was assigned to the codon of chloroacetyl lysine. The end of the base sequence set forth in SEQ ID NO: 6 is the stop codon TAA.
SEQ ID NO: 5: ACIPRGDSFAXAGGSEQKLISEEDLGGSVSGWRLFKKIS (X is chloroacetyl lysine)
SEQ ID NO: 6:

GCGTGCATCCCGCGCGGCGATTCTTTTGCATAGGCAGGCGGTTCTGAACAGAAACT
GATCAGCGAAGAAGATCTGGGTGGCTCTGTAAGTGGATGGCGATTATTCAAGAAGA
TTAGCTAA

The sequences set forth in SEQ ID Nos: 7 to 52 were designed as VKKX tags, and the sequences set forth in SEQ ID Nos: 53 to 98 were designed as base sequences encoding these sequences. Table 1-1 and Table 1-2 show the amino acid sequence and base sequence of the VKKX tag. The sequences set forth In SEQ ID NOs: 53 to 98 are examples of embodiments of the base sequences encoding SEQ ID nOs: 7 to 52, respectively, and the base sequences encoding the sequences set forth in SEQ ID nOs: 7 to 52 are not limited to the sequences set forth in SEQ ID nOs: 53 to 98.

**[Table 1-1]**

| VKKX tag | | Base sequence encoding VKKX tag | |
|---|---|---|---|
| SEQ ID NO | Amino acid sequence | SEQ ID NO | Base sequence |
| 7 | VKKI | 53 | GTTAAAAAAATA |
| 8 | VKKK | 54 | GTTAAAAAAAAA |
| 9 | VKKT | 55 | GTTAAAAAAACA |
| 10 | VKKR | 56 | GTTAAAAAACGG |
| 11 | VKKS | 57 | GTTAAAAAATCA |
| 12 | VKKH | 58 | GTTAAAAAACAT |
| 13 | VKKM | 59 | GTTAAAAAAATG |
| 14 | VKKP | 60 | GTTAAAAAACCT |
| 15 | VKKG | 61 | GTTAAAAAAGGC |
| 16 | VKKL | 62 | GTTAAAAAACTA |
| 17 | VKKV | 63 | GTTAAAAAAGTT |
| 18 | VKKE | 64 | GTTAAAAAAGAA |
| 19 | VKKA | 65 | GTTAAAAAAGCA |
| 20 | VKKD | 66 | GTTAAAAAAGAT |
| 21 | VKKC | 67 | GTTAAAAAATGT |
| 22 | VKKN | 68 | GTTAAAAAAAAT |
| 23 | VKKY | 69 | GTTAAAAAATAT |
| 24 | VKKQ | 70 | GTTAAAAAACAA |
| 25 | VKKW | 71 | GTTAAAAAATGG |
| 26 | VKKF | 72 | GTTAAAAAATTT |

**[Table 1-2]**

| VKKX tag | | Base sequence encoding VKKX tag | |
|---|---|---|---|
| SEQ ID NO | Amino acid sequence | SEQ ID NO | Base sequence |
| 27 | VKKTKT | 73 | GTTAAAAAAACAAAAACA |
| 28 | VKKTKTT | 74 | GTTAAAAAAACAAAAACAACA |
| 29 | VKKTKTD | 75 | GTTAAAAAAACAAAAACAGAT |
| 30 | VKKTKTKK | 76 | GTTAAAAAAACAAAAACAAAAAAA |
| 31 | VKKTKTKT | 77 | GTTAAAAAAACAAAAACAAAAACA |
| 32 | VKKTKTKD | 78 | GTTAAAAAAACAAAAACAAAAGAT |
| 33 | VKKTKTTK | 79 | GTTAAAAAAACAAAAACAACAAAA |
| 34 | VKKTKTTT | 80 | GTTAAAAAAACAAAAACAACAACA |
| 35 | VKKTKTDD | 81 | GTTAAAAAAACAAAAACAGATGAT |
| 36 | VKKTKTKKT | 82 | GTTAAAAAAACAAAAACAAAAAAAACA |
| 37 | VKKTKTKKD | 83 | GTTAAAAAAACAAAAACAAAAAAAGAT |
| 38 | VKKTKTKTK | 84 | GTTAAAAAAACAAAAACAAAAACAAAA |
| 39 | VKKTKTKTT | 85 | GTTAAAAAAACAAAAACAAAAACAACA |
| 40 | VKKTKTKDK | 86 | GTTAAAAAAACAAAAACAAAAGATAAA |
| 41 | VKKTKTKDT | 87 | GTTAAAAAAACAAAAACAAAAGATACA |
| 42 | VKKTKTKDD | 88 | GTTAAAAAAACAAAAACAAAAGATGAT |
| 43 | VKKTKTTKK | 89 | GTTAAAAAAACAAAAACAACAAAAAAA |
| 44 | VKKTKTTKT | 90 | GTTAAAAAAACAAAAACAACAAAAACA |
| 45 | VKKTKTTKD | 91 | GTTAAAAAAACAAAAACAACAAAAGAT |
| 46 | VKKTKTKKTK | 92 | GTTAAAAAAACAAAAACAAAAAAAACAAAA |
| 47 | VKKTKTKKDK | 93 | GTTAAAAAAACAAAAACAAAAAAAGATAAA |
| 48 | VKKTKTKTKK | 94 | GTTAAAAAAACAAAAACAAAAACAAAAAAA |
| 49 | VKKTKTKTTK | 95 | GTTAAAAAAACAAAAACAAAAACAACAAAA |
| 50 | VKKTKTKDKK | 96 | GTTAAAAAAACAAAAACAAAAGATAAAAAA |
| 51 | VKKTKTTTKK | 97 | GTTAAAAAAACAAAAACAACAACAAAAAAA |
| 52 | VKKTKTKTDTT | 98 | GTTAAAAAAACAAAAACAAAAACAGATACAACA |

A base sequence encoding a tagged fusion protein in which methionine, a VKKX tag (any of SEQ ID nOs: 7 to 52), and a fusion protein (SEQ ID NO: 5) were linked in this order was designed. In this base sequence, a base sequence (any of SEQ ID nOs: 53 to 98) encoding a VKKX tag is arranged immediately after the start codon ATG, and a base sequence encoding a fusion protein immediately after the base sequence (SEQ ID NO: 6) encoding the VKKX tag is arranged.

As a control example for evaluating the effect of the VKKX tag, a base sequence in which a base sequence (SEQ ID NO: 6) encoding a fusion protein arranged immediately after the start codon ATG was designed.

As a template DNA for expressing a fusion protein, a template DNA in which a T7 promoter sequence, a Shine-Dalgarno sequence, and a base sequence encoding a tagged fusion protein were linked from the " terminal was produced. The production of the template DNA was performed by two-step PCR.

In the first PCR step, a DNA set short in SEQ ID NO: 99 (GAAATTAATACGACTCACTATAGGGAGACCACAACGGTTTCCCTCTAGAAATAATT TTGTTTAACTTTAAGAAGGAGATATACCA) and a DNA for linking of each VKKX tag were mixed such that the concentration thereof was 1 nmol/L, and the two DNAs were linked by performing four steps of 94°C/2 minutes, 98°C/10 seconds, 43°C/30 seconds, and 68°C/15 seconds using KOD-Plus-Ver.2 (TOYOBO, KOD-211).

An example of the embodiment of the DNA for linking of each VKKX tag is the DNA having the base sequence set forth in SEQ ID NO: 100. The DNA having the base sequence set forth in SEQ ID NO: 100 is a linking DNA for a VKKX tag (VKKTKT) represented by SEQ ID NO: 27.
SEQ ID NO: 100: CGCGCGGGATGCACGC[TGTTTTTGTTTTTTTAAC]CATTGGTATATCTCCTT

In SEQ ID NO: 100, the base sequence in [ ] is a sequence complementary to the base sequence set forth in SEQ ID NO: 73 (GTTAAAAAAACAAAAACA, the base sequence encoding SEQ ID NO: 27). In the linking DNA for each VKKX tag, the base sequence in [ ] is a sequence complementary to any sequence of SEQ ID NO: 53 to 98.

Next, a forward primer (GAAATTAATACGACTCACTATAGGGAGACC) presented by SEQ ID NO: 101 and a reverse primer (CCTGCCTATGCAAAAGAATCGCCGCGCGGGATGCAC) presented by SEQ ID NO: 102 were added in each amount of 0.3 µmol/L to the linked DNA, and a DNA fragment having from the T7 promoter sequence to a part of the base sequence encoding the integrin binding peptide were produced by performing three steps of 98°C/10 seconds, 61°C/30 seconds, and 68°C/15 seconds in the presence of KOD-Plus-Ver.2 for 35 cycles. This DNA fragment was purified, diluted to 50 ng/µL, and subjected to the second PCR step.

In the second PCR step, the DNA fragment produced in the first PCR step and the DNA represented by SEQ ID NO: 103 (GGATTAGTTATTCATTACAGATCTTCTTCGCTGATCAGTTTCTGTTCAGAACCGCCT GCCTATGCAAAAGAATC) were mixed such that the concentrations thereof were 20 pg/µL, and 0.2 nmol/L, respectively, and the two DNAs were linked by performing four steps of 94°C/2 minutes, 98°C/10 seconds, 62°C/30 seconds, and 68°C/15 seconds using KOD-Plus-Ver.2. Next, a forward primer (GAAATTAATACGACTCACTATAG) represented by SEQ ID NO: 104 and a reverse primer (GGATTAGTTATTCATTACAGATC) represented by SEQ ID NO: 105 were added in each amount of 0.3 µmol/L, and the target template DNA was obtained by performing three steps of 98°C/10 seconds, 57°C/30 seconds, and 68°C/15 seconds in the presence of KOD-Plus-Ver.2 for 35 cycles. The produced template DNA was purified and diluted to 50 ng/µL.

An example of an embodiment of the template DNAis the DNAhaving the base sequence set forth in SEQ ID NO: 106. The template DNA having a base sequence set forth in SEQ ID NO: 106 is a template DNA of a tagged fusion protein to which a tag represented by SEQ ID NO: 27 is added. In the base sequence set forth in SEQ ID NO: 106, the base sequence set forth in SEQ ID NO: 73 (the base sequence encoding SEQ ID NO: 27) is arranged immediately after the start codon ATG, and the base sequence set forth in SEQ ID NO: 6 (the base sequence encoding the fusion protein) is arranged immediately after the SEQ ID NO: 73.

SEQ ID NO: 106:
GAAATTAATACGACTCACTATAGGGAGACCACAACGGTTTCCCTCTAGAAATAATTT
TGTTTAACTTTAAGAAGGAGATATACCAATG[GTTAAAAAAACAAAAACA]GCGTGC
ATCCCGCGCGGCGATTCTTTTGCATAGGCAGGCGGTTCTGAACAGAAACTGATCAG
CGAAGAAGATCTGGGTGGCTCTGTAAGTGGATGGCGATTATTCAAGAAGATTAGCT
AATGAATAACTAATCC

In SEQ ID NO: 106, the base sequence in [ ] is the sequence set forth in SEQ ID NO: 73. In the template DNA of each VKKX tag, the base sequence in [ ] is any one of the sequences set forth in SEQ ID nOs: 53 to 98.

In order to translate chloroacetyl lysine which is an unnatural amino acid, a tRNA in which the anticodon was CUA and paired with the UAG codon of mRNA was prepared. This tRNA was aminoacylated with an N-chloroacety lysine hosphoro 2'deoxyribocytidylylriboadenosine (pdCpA) ester. This aminoacyl tRNA is referred to as an Aminoacyl tRNA(1).

The cell-free biosynthesis of the polypeptide was carried out in a translation solution containing a template DNA, PUREfrex 2.0 (GeneFrontier, PF201-0.25-5), and Aminoacyl tRNA (1). 1.25 µL (62.5 ng) of the template DNA prepared to 50 ng/µL, 2.5 µL of PUREfrex 2.0 Solution I, 0.25 µL of Solution II, 0.5 µL of Solution III, and 0.5 µL of Aminoacyl tRNA (1) were mixed and mixture was subjected to a reaction at 37°C for 1 hour.

The amount of the biosynthesized polypeptide was measured by ELISA. 50 ng of Recombinant Human Integrin αVβ3 (R&D systems, 3050-AV-050) was immobilized in each well of a 96-well plate and blocked with Blocker Casein in PBS (Thermo, 37528), and then a cell-free biosynthesis solution diluted with Can Get Signal Immunoreaction Enhancer Solution I (TOYOBO, NKB-101) containing 5 mmol/L MgCl₂ was added thereto, and the mixture was subjected to a reaction at room temperature for 3 hours. After washing with 5 mmol/L MgCl₂/0.05% Tween 20-containing phosphate buffered saline (PBS), an anti-Myc antibody (Cell Signaling, 14038S) diluted with Can Get Signal Immunoreaction Enhancer Solution II (TOYOBO, NKB-101) was added thereto, and the mixture was subjected to a reaction at room temperature for 2 hours. After washing with PBS containing 5 mmol/L MgCl ₂ / 0.05% Tween 20, Super Signal ELISA Femto Substrate (Thermo, 37075) was added thereto, and the luminescence was measured. The amount of the fusion protein in the cell-free biosynthesis solution was calculated based on a calibration curve created from the fusion protein having a known concentration. The luminescence measurement was performed twice, and the average value of the amounts of the fusion protein was calculated. Table 2-1 and Table 2-2 show relative values of the biosynthesis amounts of respective fusion proteins in a case where the amount of the fusion protein of the control example was set to the reference value 1.

**[Table 2-1]**

| VKKX tag | | Biosynthesis amount of fusion protein |
|---|---|---|
| SEQ ID NO | Amino acid sequence | |
| - | - | 1 |
| 7 | VKKI | 4.40 |
| 8 | VKKK | 6.15 |
| 9 | VKKT | 5.66 |
| 10 | VKKR | 5.75 |
| 11 | VKKS | 6.05 |
| 12 | VKKH | 5.82 |
| 13 | VKKM | 1.94 |
| 14 | VKKP | 2.23 |
| 15 | VKKG | 2.39 |
| 16 | VKKL | 2.71 |
| 17 | VKKV | 2.90 |
| 18 | VKKE | 2.94 |
| 19 | VKKA | 3.01 |
| 20 | VKKD | 3.05 |
| 21 | VKKC | 3.14 |
| 22 | VKKN | 3.17 |
| 23 | VKKY | 3.30 |
| 24 | VKKQ | 3.52 |
| 25 | VKKW | 3.52 |
| 26 | VKKF | 3.69 |

**[Table 2-2]**

| VKKX tag | | Biosynthesis amount of fusion protein |
|---|---|---|
| SEQ ID NO | Amino acid sequence | |
| 27 | VKKTKT | 4.94 |
| 28 | VKKTKTT | 6.28 |
| 29 | VKKTKTD | 6.09 |
| 30 | VKKTKTKK | 9.01 |
| 31 | VKKTKTKT | 9.08 |
| 32 | VKKTKTKD | 8.04 |
| 33 | VKKTKTTK | 6.54 |
| 34 | VKKTKTTT | 6.69 |
| 35 | VKKTKTDD | 5.75 |
| 36 | VKKTKTKKT | 10.50 |
| 37 | VKKTKTKKD | 11.45 |
| 38 | VKKTKTKTK | 8.42 |
| 39 | VKKTKTKTT | 7.22 |
| 40 | VKKTKTKDK | 8.49 |
| 41 | VKKTKTKDT | 6.27 |
| 42 | VKKTKTKDD | 5.35 |
| 43 | VKKTKTTKK | 9.45 |
| 44 | VKKTKTTKT | 7.83 |
| 45 | VKKTKTTKD | 7.83 |
| 46 | VKKTKTKKTK | 9.94 |
| 47 | VKKTKTKKDK | 8.90 |
| 48 | VKKTKTKTKK | 10.47 |
| 49 | VKKTKTKTTK | 7.44 |
| 50 | VKKTKTKDKK | 7.78 |
| 51 | VKKTKTTTKK | 6.29 |
| 52 | VKKTKTKTDTT | 4.33 |

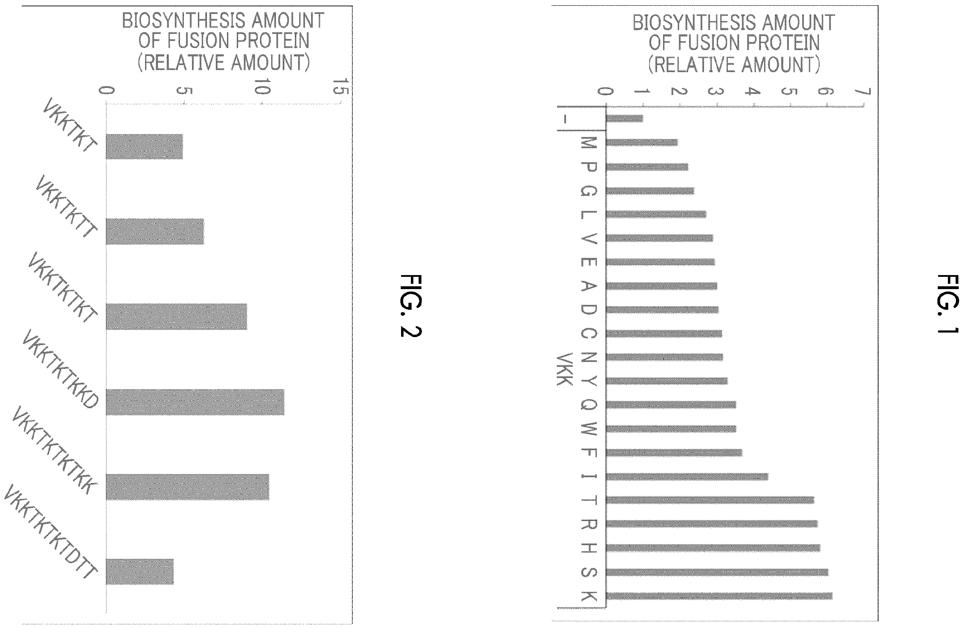

The biosynthesis amount of the fusion protein was increased by 1.9 times to 6.2 times by the tags represented by SEQ ID nOs: 7 to 26 consisting of 4 amino acids. Fig. 1 is a graph showing a biosynthesis amount of a fusion protein for tags having SEQ ID nOs: 7 to 26, in which the tags are arranged in the order of the biosynthesis amounts of the fusion proteins. By each of the tags of VKKI, VKKT, VKKR, VKKH, VKKS, and VKKK, the biosynthesis amount of the fusion protein was increased by 4.4 times to 6.2 times. That is, as the tag consisting of 4 amino acids, a tag in which I (Ile), T (Thr), R (Arg), H (His), S (Ser), or K (Lys) is arranged immediately after VKK is dominant.

The biosynthesis amount of the fusion protein was increased by 4.9 times by the tags consisting of VKKTKT (SEQ ID NO: 27).

By arranging one or more residues of T, K, and D following VKKTKT and extending the whole length of the tag to 10 amino acids (that is, SEQ ID NOs: 28 to 51), the biosynthesis amount of the fusion protein is 5.4 times to 11.5 times. In the example in which the whole length of the tag was 11 amino acids (that is, SEQ ID NO: 52), the increase in the biosynthesis amount of the fusion protein was 4.3 times. Fig. 2 is a graph showing a biosynthesis amount of a fusion protein for the tags having SEQ ID NOs: 27, 28, 31, 37, 48, and 52.

That is, the superiority of the tag in which 0 to 4 residues of T (Thr), K (Lys), and D (Asp) were arranged following VKKTKT (that is, the whole length of the tag was 10 amino acids or less) was shown.

### <Example 2: Examination of base sequence encoding tag>

Regarding the effect of increasing the biosynthesis amount of the polypeptide by the tag represented by SEQ ID NO: 27 (hereinafter, referred to as a VKKTKT tag), the influence of the GC content of the base sequence encoding the tag was examined. As a control for comparison, a SKIC tag, which has a known effect of increasing the biosynthesis amount of a polypeptide, was selected (SEQ ID NO: 107, the SKIK tag is disclosed in WO2016/204198A). The polypeptide to be biosynthesized is the fusion protein containing the integrin binding peptide, as in Example 1.

Three types of each of the base sequence encoding the VKKTKT tag and the base sequence encoding the SKIK tag were designed. Table 3 shows the amino acid sequence and base sequence of the VKKX tag.

**[Table 3]**

| Tag | | Base sequence encoding tag | | |
|---|---|---|---|---|
| SEQ ID NO | Amino acid sequence | SEQ ID NO | Base sequence | GC content [%] |
| 107 | SKIK | 108 | AGTAAAATTAAA | 8 |
| | | 109 | AGTAAGATTAAG | 25 |
| | | 110 | TCGAAGATCAAG | 42 |
| 27 | VKKTKT | 73 | GTTAAAAAAACAAAAACA | 17 |
| | | 111 | GTTAAGAAAACCAAGACA | 33 |
| | | 112 | GTGAAGAAGACCAAGACC | 50 |

A base sequence encoding a tagged fusion protein in which methionine, a tag (SEQ ID NO: 107 or 27), and a fusion protein (SEQ ID NO: 5) were linked in this order was designed. In this base sequence, a base sequence (any of SEQ ID NOs: 108 to 112, or 73) encoding a tag is arranged immediately after the start codon ATG, and a base sequence encoding a fusion protein immediately after the base sequence (SEQ ID NO: 6) encoding the tag is arranged.

A template DNA was produced by two-step PCR in the same manner as in Example 1. In the first PCR step, the DNA represented by SEQ ID NO: 99 and the DNA for linking designed for each tag (the example of the embodiment was SEQ ID NO: 100) were mixed such that the concentration thereof was 1 nmol/L, and the two DNAs were linked by performing four steps of 94°C/2 minutes, 98°C/10 seconds, 43°C/30 seconds, and 68°C/15 seconds using the KOD-Plus-Ver.2 (TOYOBO, KOD-211). Next, a forward primer represented by SEQ ID NO: 101 and a reverse primer represented by SEQ ID NO: 102 were added in each amount of 0.3 µmol/L, and a DNA fragment having from the T7 promoter sequence to a part of the sequence encoding the integrin binding peptide was produced by performing three steps of 98°C/10 seconds, 61°C/30 seconds, and 68°C/15 seconds in the presence of KOD-Plus-Ver.2 for 35 cycles. This DNA fragment was purified, diluted to 50 ng/µL, and subjected to the second PCR step. In the second PCR step, the DNA fragment produced in the first PCR step and the DNA represented by SEQ ID NO: 103 were mixed such that the concentrations thereof were 20 pg/µL and 0.2 nmol/L, respectively, and the two DNAs were linked by performing four steps of 94°C/2 minutes, 98°C/10 seconds, 62°C/30 seconds, and 68°C/15 seconds using the KOD-Plus-Ver.2. Next, 0.3 µmol/L of each of a forward primer represented by SEQ ID NO: 104 and a reverse primer represented by SEQ ID NO: 105 was added thereto, three steps of 98°C/10 seconds, 57°C/30 seconds, and 68°C/15 seconds were carried out in the presence of 2 for 35 cycles to obtain a target template DNA (an embodiment example was SEQ ID NO: 106). The produced template DNA was purified and diluted to 50 ng/µL.

In the same manner in Example 1, the fusion protein was expressed by the cell-free peptide synthesis system and quantified by ELISA. The measurement was performed twice, and the average value of the amounts of the fusion protein was calculated. Table 4 shows relative values of the amounts of respective fusion proteins in a case where the amount of the fusion protein of the control example was set to the reference value 1. As in Example 1, the control example of the reference value 1 has a form in which the base sequence (SEQ ID NO: 6) encoding the fusion protein is arranged immediately after the start codon ATG.

**[Table 4]**

| Tag | | Base sequence encoding tag | | Biosynthesis amount of fusion protein |
|---|---|---|---|---|
| SEQ ID NO | Amino acid sequence | SEQ ID NO | GC content [%] | |
| - | - | - | - | 1 |
| 107 | SKIK | 108 | 8 | 3.79 |
| | | 109 | 25 | 4.18 |
| | | 110 | 42 | 1.46 |
| 27 | VKKTKT | 73 | 17 | 4.94 |
| | | 111 | 33 | 3.83 |
| | | 112 | 50 | 3.77 |

The expression level of the fusion protein was increased by the SKIK tag, and the increase rate was 1.5 times to 4.2 times. An influence of the GC content of the base sequence encoding the SKIK tag was observed on the degree of increase in the expression level.

The expression level of the fusion protein was increased by the VKKTKT tag, and the increase rate was 3.8 times to 4.9 times. An influence of the GC content of the base sequence encoding the VKKTKT tag was hardly affected on the degree of increase in the expression level. That is, by the VKKTKT tag, the expression level of the fusion protein was increased regardless of the encoding base sequence.

### <Example 3: Examination of translation condition>

Regarding the effect of increasing the biosynthesis amount of the polypeptide by the VKKX tag represented by SEQ ID NOs: 27 to 52, it was examined whether the effect could be exhibited even in a case where the incubation time for biosynthesis was shortened. As a comparative control, a SKIK tag represented by SEQ ID NO: 107 was selected. The polypeptide to be biosynthesized is the fusion protein containing the integrin binding peptide, as in Example 1.

Using a template DNA including DNA sequences (SEQ ID NOs: 73 to 98) encoding VKKX tags represented by SEQ ID NOs: 27 to 52 produced in Example 1, and a DNA sequence (SEQ ID NO: 110) encoding a SKIK tag represented by SEQ ID NO: 107 produced in Example 2, the biosynthesis reaction was performed in the same manner as in Example 1 by setting the reaction time to 10 minutes. In the same manner in Example 1, the fusion protein was expressed by the cell-free peptide synthesis system and quantified by ELISA. The measurement was performed twice, and the average value of the amounts of the fusion protein was calculated. Table 5 shows relative values of the amounts of respective fusion proteins in a case where the amount of the fusion protein of the control example was set to the reference value 1. As control example used in Example 1, the control example of the reference value 1 has a form in which the base sequence (SEQ ID NO: 6) encoding the fusion protein is arranged immediately after the start codon ATG.

**[Table 5]**

| VKKX tag | | Biosynthesis amount of fusion protein |
|---|---|---|
| SEQ ID NO | Amino acid sequence | |
| 27 | VKKTKT | 12.81 |
| 28 | VKKTKTT | 16.65 |
| 29 | VKKTKTD | 15.90 |
| 30 | VKKTKTKK | 24.42 |
| 31 | VKKTKTKT | 15.48 |
| 32 | VKKTKTKD | 20.56 |
| 33 | VKKTKTTK | 8.17 |
| 34 | VKKTKTTT | 8.59 |
| 35 | VKKTKTDD | 8.49 |
| 36 | VKKTKTKKT | 13.13 |
| 37 | VKKTKTKKD | 19.84 |
| 38 | VKKTKTKTK | 16.95 |
| 39 | VKKTKTKTT | 7.57 |
| 40 | VKKTKTKDK | 10.77 |
| 41 | VKKTKTKDT | 28.80 |
| 42 | VKKTKTKDD | 14.95 |
| 43 | VKKTKTTKK | 23.35 |
| 44 | VKKTKTTKT | 7.18 |
| 45 | VKKTKTTKD | 10.61 |
| 46 | VKKTKTKKTK | 23.47 |
| 47 | VKKTKTKKDK | 15.53 |
| 48 | VKKTKTKTKK | 36.08 |
| 49 | VKKTKTKTTK | 8.75 |
| 50 | VKKTKTKDKK | 35.42 |
| 51 | VKKTKTTTKK | 15.28 |
| 52 | VKKTKTKTDTT | 9.15 |
| 107 | SKIK | 1.27 |

Even in a case where the incubation time of the biosynthesis reaction was set to 10 minutes, the expression level of the fusion protein was increased by the VKKX tag, and the increase rate was 7.2 times to 36 times. On the other hand, the expression level of the fusion protein was also increased by the SKIK tag, but the increase rate was 1.3 times. That is, the VKKX tag showed the effect of increasing the expression level even for a shorter biosynthesis reaction time.

### <Example 4: Examination of peptide length>

Regarding the effect of increasing the biosynthesis amount of the polypeptide by the tag (VKKTKT tag) represented by SEQ ID NO: 27, the influence of the length of the polypeptide to be synthesized was examined. The polypeptide to be biosynthesized is a fusion protein containing a partial sequence of dihydrofolate reductase (DHFR) and a luminescent (HiBiT) tag.

A fusion protein in which a partial sequence of DHFR and a HiBiT tag were linked in this order was designed. Gly-Gly-Ser was inserted as a spacer between the partial sequence of DHFR and the HiBiT tag. The amino acid sequences of these fusion proteins are set forth in SEQ ID NOs: 113 and 114, and these fusion proteins are a fusion protein (80 amino acid length) of the amino acids ranging to the amino acid at position 66 from the N-terminal of DHFR with a HiBiT tag and a fusion protein (100 amino acid length) of the amino acids ranging to the amino acid at position 86 from the N-terminal of DHFR with a HiBiT tag. The base sequences encoding these fusion proteins are SEQ ID NOs: 115 and 116.
SEQ ID NO: 113: 
VGSLNCIVAVSQNMGIGKNGDLPWPPLRNEFRYFQRMTTTSSVEGKQNLVIMGKKTW
FSIPEKNRPGGSVSGWRLFKKIS
SEQ ID NO: 114: 
VGSLNCIVAVSQNMGIGKNGDLPWPPLRNEFRYFQRMTTTSSVEGKQNLVIMGKKTW
FSIPEKNRPLKGRINLVLSRELKEPPQGAGGSVSGWRLFKKIS
SEQ ID NO: 115: 
GTTGGATCCTTGAACTGCATCGTAGCTGTGAGCCAAAACATGGGAATTGGGAAGAA
CGGCGATTTACCCTGGCCACCGTTGCGGAATGAATTCCGCTATTTTCAGCGTATGAC
CACCACAAGTTCGGTGGAAGGGAAACAGAATCTGGTGATCATGGGCAAGAAAACG
TGGTTTAGCATTCCGGAGAAGAATCGTCCTGGTGGCTCTGTAAGTGGATGGCGATT
ATTCAAGAAGATTAGC
SEQ ID NO: 116:

GTTGGATCCTTGAACTGCATCGTAGCTGTGAGCCAAAACATGGGAATTGGGAAGAA
CGGCGATTTACCCTGGCCACCGTTGCGGAATGAATTCCGCTATTTTCAGCGTATGAC
CACCACAAGTTCGGTGGAAGGGAAACAGAATCTGGTGATCGGCAAGAAAACGTG
GTTTAGCATTCCGGAGAAGAATCGTCCTCTGAAAGGCCGTATCAACCTCGTTCTGT
CACGCGAACTGAAAGAGCCTCCACAAGGTGCAGGTGGCTCTGTAAGTGGATGGCG

ATTATTCAAGAAGATTAGC

Abase sequence encoding a tagged fusion protein in which methionine, a VKKTKT tag (SEQ ID NO: 27) or a SKIK tag (SEQ ID NO: 107), and a fusion protein (any of SEQ ID NO: 113 or 114) were linked in this order was designed. In this base sequence, a base sequence (SEQ ID NO: 73 or 110) encoding a VKKTKT tag or a SKIK tag is arranged immediately after the start codon ATG, and a base sequence (any of SEQ ID NO: 115 or 116) encoding a fusion protein is arranged immediately after the base sequence encoding the VKKTKT tag or the SKIK tag.

As a control example for evaluating the effect of the VKKTKT tag or the SKIK tag, a base sequence in which a base sequence (any of SEQ ID NO: 115 or 116) encoding each of fusion protein arranged immediately after the start codon ATG was designed.

As a template DNA for expressing a fusion protein, a template DNA in which a T7 promoter sequence, a Shine-Dalgarno sequence, and a base sequence encoding a tagged fusion protein were linked from the " terminal was produced. The template DNA containing the sequences set forth in SEQ ID NOs: 115 and 116 was produced by three-step PCR.

In the first PCR step, the DNA encoding DHRF represented by SEQ ID NO: 117, the forward primer for adding the VKKTKT tag represented by SEQ ID NO: 118 or the forward primer for adding the SKIK tag represented by SEQ ID NO: 119, and the reverse primer represented by SEQ ID NO: 120 were added in each amount of 0.3 µmol/L, and a DNAfragment having the whole length of the base sequence encoding DHFR to which the VKKTKT tag or the SKIK tag was added was produced by performing three steps of 98°C/10 seconds, 61°C/30 seconds, and 68°C/15 seconds in the presence of KOD-Plus-Ver.2 for 35 cycles. This DNA fragment was purified, diluted to 50 ng/µL, and subjected to the second PCR step.
SEQ ID NO: 117: 
GTTGGATCCTTGAACTGCATCGTAGCTGTGAGCCAAAACATGGGAATTGGGAAGAA
CGGCGATTTACCCTGGCCACCGTTGCGGAATGAATTCCGCTATTTTCAGCGTATGAC
CACCACAAGTTCGGTGGAAGGGAAACAGAATCTGGTGATCATGGGCAAGAAAACG
TGGTTTAGCATTCCGGAGAAGAATCGTCCTCTGAAAGGCCGTATCAACCTCGTTCT
GTCACGCGAACTGAAAGAGCCTCCACAAGGTGCACACTTCCTTAGCCGCAGTCTT
GATGATGCCCTGAAACTGACTGAACAGCCCGAATTAGCGAACAAAGTCGATATGGT
CTGGATTGTAGGTGGCTCTTCCGTGTACAAAGAAGCGATGAATCATCCGGGTCATCT
GAAACTGTTTGTTACGCGCATTATGCAGGACTTTGAATCGGATACCTTCTTTCCGGA
AATTGACCTCGAGAAATACAAACTGTTACCGGAATATCCGGGTGTTCTGTCTGATGT
 GCAGGAAGAGAAAGGCATCAAATACAAATTCGAAGTCTATGAGAAGAACGAC
SEQ ID NO: 118:
   AAGGAGATATACCAATGGTTAAAAAAACAAAAACAGTTGGATCCTTGAACTGCAT
SEQ ID NO: 119:
   AAGGAGATATACCAATGTCGAAGATCAAGGTTGGATCCTTGAACTGCAT
SEQ ID NO: 120: TTAGTCGTTCTTCTCATAGA

In the second PCR step, the DNA fragment produced in the first PCR step and the DNA represented by SEQ ID NO: 99 were mixed such that the concentration thereof was 1 nmol/L, and the two DNAs were linked by performing four steps of 94°C/2 minutes, 98°C/10 seconds, 43°C/30 seconds, and 68°C/15 seconds using the KOD-Plus-Ver.2 (TOYOBO, KOD-211).

Next, a forward primer represented by SEQ ID NO: 101 and any of reverse primers represented by SEQ ID NO: 121 or 122 were added to the linked DNA in each amount of 0.3 µmol/L, and a DNA fragment having a part of the base sequence encoding the DHFR from the T7 promoter sequence was produced by performing three steps of 98°C/10 seconds, 61°C/30 seconds, and 68°C/15 seconds in the presence of KOD-Plus-Ver.2 for 35 cycles. This DNA fragment was purified, diluted to 50 ng/µL, and subjected to the third PCR step.
SEQ ID NO: 121: AGGACGATTCTTCTCCGGAAT
SEQ ID NO: 122: TGCACCTTGTGGAGGCTCTTT

In the third PCR step, the DNA fragment produced in the second PCR step and the DNA represented by SEQ ID NO: 123 or 124 were mixed such that the concentration thereof was 1 nmol/L, and the two DNAs were linked by performing four steps of 94°C/2 minutes, 98°C/10 seconds, 43°C/30 seconds, and 68°C/15 seconds using the KOD-Plus-Ver.2 (TOYOBO, KOD-211).
SEQ ID NO: 123: 
GGATTAGTTATTCATTAGCTAATCTTCTTGAATAATCGCCATCCACTTACAGAGCCAC
CAGGACGATTCTTCTCCGGAAT
SEQ ID NO: 124:

GGATTAGTTATTCATTAGCTAATCTTCTTGAATAATCGCCATCCACTTACAGAGCCAC
CTGCACCTTGTGGAGGCTCTTT

Next, a forward primer represented by SEQ ID NO: 101 and a reverse primer represented by SEQ ID NO: 105 were added to the linked DNA in each amount of 0.3 µmol/L, and a DNA fragment having the base sequence encoding the fusion protein from the T7 promoter sequence were produced by performing three steps of 98°C/10 seconds, 61°C/30 seconds, and 68°C/15 seconds in the presence of KOD-Plus-Ver.2 for 35 cycles. This DNA fragment was purified and diluted to 50 ng/µL.

The fusion protein was expressed using a cell-free peptide synthesis system similar to the cell-free peptide synthesis system used in Example 1 by setting the incubation time of biosynthesis to 10 minutes. The obtained fusion protein was quantified using Nano Glo HiBiT Lytic Detection System (Promega, N3040). The measurement was performed twice, and the average value of the amounts of the fusion protein was calculated. Table 6 shows relative values of the amounts of respective fusion proteins in a case where the amount of the fusion protein of the control example was set to the reference value 1. The control example of the reference value 1 has a form in which the base sequence (SEQ ID NO: 115 or 116) encoding the fusion protein is arranged immediately after the start codon ATG.

**[Table 6]**

| Tag | | Fusion protein | | Biosynthesis amount of fusion protein |
|---|---|---|---|---|
| SEQ ID NO | Amino acid sequence | SEQ ID NO | Amino acid length | |
| - | - | 113 | 80 | 1 |
| 27 | VKKTKT | 113 | 80 | 1.67 |
| 107 | SKIK | 113 | 80 | 1.13 |
| - | - | 114 | 100 | 1 |
| 27 | VKKTKT | 114 | 100 | 1.24 |
| 107 | SKIK | 114 | 100 | 0.73 |

Generally, the translation promoting effect decreases as the length of the amino acid increases. However, the VKKTKT tag exhibits a translation promoting effect also for a fusion protein having a length of 80 to 100 amino acids, and the promoting effect is 1.2 times to 1.7 times. This effect was more excellent than that of the SKIK tag.

### <Example 5: Evaluation of binding ability of tagged peptide>

It is conceived that since the polypeptide can be obtained at a high yield by the addition of the tag sequence according to the present disclosure, the Emax value and the EC50 value, which are important indicators of the binding ability, can be accurately measured by the binding evaluation of the high-concentration polypeptide. That is, it is considered that the biosynthesis method of a polypeptide using the tag sequence according to the present disclosure and the produced polypeptide can be used for evaluation of binding performance. Therefore, it was examined whether the binding evaluation could be carried out using the polypeptide to which the VKKTKT tag represented by SEQ ID NO: 27 was added.

Using a cell-free peptide synthesis system derived from Escherichia coli, a tag sequence set forth in SEQ ID NO: 27, and a template DNA sequence (SEQ ID NO: 106) encoding from an integrin binding peptide to a HiBiT tag, an integrin binding peptide was biosynthesized in a high yield, and the integrin binding ability of the obtained peptide was measured by ELISA evaluation of the amount of peptide binding to integrin at each peptide concentration. The details of the production method of template DNA, the method of cell-free peptide biosynthesis, and the ELISA operation are the same as in Example 1. In the measurement of the peptide concentration, a peptide diluted 5,000 times with Can Get Signal Immunoreaction Enhancer Solution I (TOYOBO) containing 5 mmol/L MgCh was measured using Nano Glo HiBiT Lytic Detection System (Promega) and chemical synthetic peptide (SEQ ID NO: 125) for calibration curve having a known concentration according to the standard protocol of Nano Glo HiBiT Lytic Detection System.
SEQ ID NO: 125: EQKLISEEDLGGSVSGWRLFKKIS

In addition, as a comparative control, the binding ability (Kd value) of the integrin binding peptide was measured by the SPR method. An integrin binding peptide (SEQ ID NO: 126) was chemically synthesized and immobilized on a CM5 chip (Cytiva, BR100530) by an amine coupling method. The binding ability of the immobilized integrin binding peptide to Recombinant Human Integrin αVβ3 (R&D systems, 3050-AV-050) was measured using Biacore T-100 (Cytiva). As the measurement buffer, an HBS-N buffer (Cytiva, BR100670) in which MgCl₂ was added such that the final concentration was 5 mmol/L, and integrin was added thereto at a concentration of 10, 30, 100, or 300 nmol/L for 10 minutes. The CM5 chip was regenerated and used by repeating the addition of 500 mmol/L EDTA for 10 minutes twice.
SEQ ID NO: 126: CEPRGDNYRXGGSKKK (X is chloroacetyl lysine)

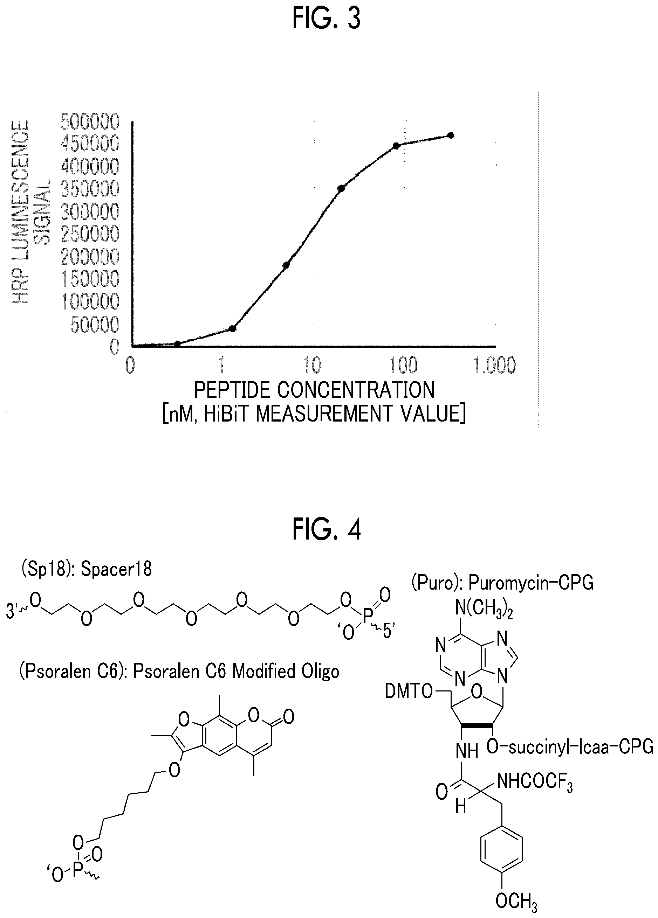

The EC50 value of the present integrin binding peptide determined from the result of the luminescence measurement of ELISA and the amount of the integrin binding peptide measured using the Nano Glo HiBiT Lytic detection system was 7.7 nM (Fig. 3), and was coincident with the Kd value (substantially the same as the EC50 value) of 7.6 nM, which was measured by the SPR method. The present results show that since a high yield of peptide can be acquired by biosynthesis using the tag sequence and binding evaluation of the peptide having a high concentration can be performed, the Emax value and the EC50 value, which is an important indicator of the binding ability, can be accurately measured.

### <Example 6: integrin binding peptide screening from VKKX tag-containing nucleic acid display library>

It was examined that a polypeptide which binds to a target substance was able to be obtained from a VKKX tag-containing nucleic acid display library. Specifically, integrin was used as a target substance, and it was examined whether or not the extracted group of polypeptides included a polypeptide having a known binding motif (amino acid sequence RGD).

The sequences set forth in SEQ ID NOs: 127 and 128 were used as the VKKX tag-containing nucleic acid display library. In these VKKX tag-containing nucleic acid display libraries, translation starts from the start codon (ATG) at the positions 86 to 88 from the 5' terminal, bases (GTTAAGAAAACAAAAACA) at the positions 89 to 106 correspond to the VKKX tag, bases (NNNTGT(NNN)₈TAGNNN (NNN is a trimer oligonucleotide, where N's each independently represent A, T, G, or C)) at the positions 107 to 142 correspond to the random sequence, and bases after the position 143 correspond a binding site of puromycin linker and a stop codon. Since the triplet TAG in the present random sequence is assigned to the codon of the chloroacetyl lysine, the polypeptide encoded by the present random sequence is, as in Example 1, a cyclic polypeptide obtained by spontaneously forming a thioether bond between a thiol group of cysteine and a chloroacetyl group of the chloroacetyl lysine. In the nucleic acids having the sequences set forth in SEQ ID NOs: 127 and 128, the trimer oligonucleotide represented by NNN is an equal amount mixture of trimer nucleotides corresponding to 18 types of codons shown in Table 7, in which one type of codon is assigned to one type of amino acid.

**[Table 7]**

| Amino acid (one-letter notation) | Assigned codon | Amino acid (one-letter notation) | Assigned codon |
|---|---|---|---|
| A | GCT | N | AAC |
| I | ATC | Q | CAG |
| L | CTG | R | CGT |
| V | GTT | H | CAT |
| F | TTC | K | AAA |
| W | TGG | D | GAC |
| Y | TAC | E | GAA |
| S | TCT | G | GGT |
| T | ACT | P | CCG |

SEQ ID NO: 127: GAAATTAATACGACTCACTATAGGGAGACCACAACGGTTTCCCTCTAGAAATAATTT TGTTTAACTTTAAGAAGGAGATATACATATGGTTAAGAAAACAAAAACANNNTGT(N NN)₈TAGNNNGGTGGCTCTGGCGGTAGCAGGACGGGGGGCGGCGGGGGGTAAATA AATAAGCTTGAGTAT (NNN is a trimer oligonucleotide, where N's each independently represent A, T, G, or C)
SEQ ID NO: 128: GAAATTAATACGACTCACTATAGGGAGACCACAACGGTTTCCCTCTAGAAATAATTT TGTTTAACTTTAAGAAGGAGATATACATATGGTTAAGAAAACAAAAACANNNTGT(N NN)₈TAGNNNGGTGGCTCTGGCGGTAGCGGCGGGGGGCGGGAGGCGGGAATAAATA AGCTTGAGTAT (NNN is a trimer oligonucleotide, where N's each independently represent A, T, G, or C)

**[Table 8]**

| Condition | SEQ ID NO of library | SEQ ID NO of DNA for library production | SEQ ID NO of puromycin linker | Presence or absence of UV irradiation | SEQ ID NO of DNA for amplification | Existence rate of RGD motif-containing peptide |
|---|---|---|---|---|---|---|
| 1 | 127 | 131 | 133 | Present | 131 | 93% |
| 2 | 127 | 131 | 134 | Present | 131 | 96% |
| 3 | 127 | 131 | 135 | Absent | 131 | 93% |
| 4 | 128 | 132 | 136 | Absent | 132 | 87% |

VKKX tag-containing nucleic acid display libraries represented by SEQ ID NOs: 127 and 128 were produced by performing overlap extension PCR. Specifically, regarding the libraries represented by SEQ ID NOs: 127 and 128, a target VKKX tag-containing nucleic acid display library was produced by mixing three types of DNAs, the DNA represented by SEQ ID NO: 129, the DNA represented by SEQ ID NO: 130, and the DNA represented by any of SEQ ID NO: 131 or 132 described in Table 8, in combinations listed in Table 8 such that the concentrations thereof were 3 µmol/L, 1 µmol/L, and 1 µmol/L, respectively, repeatedly performing, after 98°C/30 seconds, three steps of 98°C/10 second, 60°C/10 seconds, and 72°C/10 seconds for 7 cycles in the presence of Platinum^{™} SuperFi II DNA Polymerase (Thermo, 12361010), and finally performing the treatment of 72°C/5 minutes, thereby linking the three DNAs. The produced VKKX tag-containing nucleic acid display library was purified and diluted to 2 ng/µL. In the DNA set forth in SEQ ID NO: 130, the trimer oligonucleotide represented by NNN is an equal amount mixture of trimer nucleotides corresponding to 18 types of codons shown in Table 7, in which one type of codon is assigned to one type of amino acid.
SEQ ID NO: 129: 
GAAATTAATACGACTCACTATAGGGAGACCACAACGGTTTCCCTCTAGAAATAATTT
TGTTTAACTTTAAGAAGGAGATATACATATGGTTAAAAAAACAAAAAC
SEQ ID NO: 130: AAGAAGGAGATATACATATGGTTAAAAAAACAAAAACANNNTGT(NNN)₈TAGNNNG GCGGTTCTGGCGGTAGC (NNN is a trimer oligonucleotide, where N's each independently represent A, T, G, or C)
SEQ ID NO: 131: 
ATACTCAAGCTTATTTATTTATTACCCCCCGCCGCCCCCCGTCCTGCTACCGCCAGAA
CCACC
SEQ ID NO: 132:

ATACTCAAGCTTATTTATTTATCCCGCCTCCCGCCCCCCGTCCGCTACCGCCAGAAC
CACC

Using Recombinant Human Integrin αVβ3 (R&D systems, 3050-AV-050) as a target substance (integrin), immobilization (integrin concentration during immobilization: 1 µg/µL) was performed on magnetic beads (NHS Mag Sepharose, Cytiva, 28951380) according to a protocol designated by the manufacturer (Cytiva).

After repeating a step of bringing a VKKX tag-containing nucleic acid display library into contact with a target substance (magnetic bead-immobilized integrin) and performing incubation, for a total of 4 rounds, the base sequence of the nucleic acid of the nucleic acid-polypeptide conjugate was identified using a sequencer.

The specific procedure of each round is as follows. First, the produced VKKX tag-containing nucleic acid display libraries (SEQ ID NOs: 127 and 128) were reacted at 37°C for 30 minutes in the presence of T7 RNA Polymerase (TaKaRa, 2540A) to produce library transcriptions. This DNA fragment was purified and diluted to 10 µM.

Next, in a TBS buffer (1.25 mM Tris, 25 mM NaCl, pH 7.5), the library transcription (final concentration: 5 µM) and the puromycin linker (final concentration: 10 µM) shown in the column of SEQ ID NOs: 133 to 136 of Table 9 were mixed in the combination of Table 8 and then reacted at 95°C for 5 minutes. The mixed solution containing the puromycin linker having an ultraviolet crosslinking compound (Psoralen C6) is irradiated with 10 J UV (365 nm) on ice, thereby producing a complex of the library transcription product and the puromycin linker.

**[Table 9]**

| SEQ ID NO | Nucleic acid sequence |
|---|---|
| 133 | 5'-(PsoralenC6)-TE1CCCCCCGCCGCCCCCCGTCCT(Sp18)-(Sp18)-(Sp18)-(Sp18)-CC-(Puro)-3' |
| 134 | 5'-(PsoralenC6)-UACCCCCCGCCGCCCCCCGUCCU-(Sp18)-(Spl8)-(Sp18)-(Sp18)-CC-(Puro)-3' |
| 135 | 5'-UACCCCCCGCCGCCCCCCGUCCU-(Sp18)-(Spl8)-(Sp18)-(Sp18)-CC-(Puro)-3' |
| 136 | 5'-CCCGCCUCCCGCCCCCCGUCC-(Sp18)-(Sp18)-(Sp18)-(Sp18)-CC-(Puro)-3' |

| | |
|---|---|
| *See Fig. 4 for the structures of Psoralen C6, Sp18, and Puro. *The underlined nucleotides represent that 2'OH of RNA has been formed into a 2'OMe form, and the nucleotides that are not underlined represent unmodified DNA. | |

In the sequence listing, SEQ ID NOs: 133 to 136 indicate only the sequence of the body part (that is, the portion not including the linker and CC).

The complex of the library transcription and the puromycin linker were translated in a translation solution including PUREfrex 2.0 (GeneFrontier, PF201-0.25-5) and aminoacyl tRNA (the same as in Example 1). 10 µL of the complex, 4.5 µL of PUREfrex 2.0 Solution I, 0.75 µL of Solution II, 0.75 µL of Solution III, and 3 µL of Aminoacyl tRNA (1) were mixed and reacted at 37°C for 30 minutes to produce mRNA-polypeptide conjugate.

15 µL of the translation product and the DNA (final concentration of 10 µM) represented by SEQ ID NO: 137 were mixed in the presence of ReverTra Ace (TOYOBO, TRT-101) (reaction volume of 37.5 µL) and reacted at 37°C for 30 minutes to perform reverse transcription and to produce a cDNA-mRNA-polypeptide conjugate.
SEQ ID NO: 137: GCTACCGCCAGAACCACC

Next, 22.5 µL of the reverse transcription product was mixed with 10 µL of the magnetic bead-immobilized integrin in an HBS buffer (10 mM Hepes, 150 mM NaCl, 5 mM MgCl₂, pH 7.5) (reaction volume of 37.5 µL), and reacted at room temperature for 45 minutes. The magnetic beads were then washed 3 times with 100 µL of an HBS buffer to extract a cDNA-mRNA-polypeptide conjugate that binds to integrin. The extracted cDNA-mRNA-polypeptide conjugate was amplified by the following two-step PCR.

In the first PCR step, an amplification product was obtained by mixing 25 µL of the cDNA-mRNA-polypeptide conjugate with the DNA represented by SEQ ID NO: 138 (final concentration of 0.5 µM) and the DNA represented by SEQ ID NO: 139 (final concentration of 0.5 µM) (reaction volume of 50 µL), repeatedly performing, after 98°C/30 seconds, three steps of 98°C/10 seconds, 60°C/10 seconds, and 72°C/10 seconds for 6 to 15 cycles in the presence of Platinum^{™} SuperFi II DNA Polymerase (Thermo, 12361010), and finally performing the treatment of 72°C/5 minutes. The amplification product was purified and diluted to 20 nM.
SEQ ID NO: 138: GGAGATATACATATGGTTAAGAAAACAAAAAC
SEQ ID NO: 139: CTGCTACCGCCAGAACCACC

In the second PCR step, DNA having the same sequence as the original VKKX tag-containing nucleic acid display library excluding the random sequence was obtained by mixing the amplification product of the first PCR step (final concentration of 10 nM) with the DNA represented by SEQ ID NO: 129 (final concentration of 0.5 µM) and the DNA represented by any of SEQ ID NO: 131 or 132 (final concentration of 0.5 µM) described in Table 8, repeatedly performing, after 98°C/30 seconds, three steps of 98°C/10 seconds, 60°C/10 seconds, and 72°C/10 seconds for 6 cycles in the presence of Platinum^{™} SuperFi II DNA Polymerase (Thermo, 12361010), and finally performing the treatment of 72°C/5 minutes. The produced VKKX tag-containing nucleic acid display library was purified, diluted to 2 ng/µL, and used in the subsequent rounds.

The base sequence product of the fourth round in the first PCR step was identified according to the standard protocol of Illumina, Inc. using MiSeq (manufactured by Illumina, Inc.) and MiSeq Reagent Kit V2 (300 cycles) (Illumina, MS-102-2022). Table 8 shows the proportion of polypeptides having a known integrin binding motif (amino acid sequence RGD) in the identified polypeptide group.

The present result shows that a polypeptide binding to a target substance can be obtained from a VKKX tag-containing nucleic acid display library.

The disclosure of Japanese Patent Application No. 2021-157186 filed on September 27, 2021 is incorporated herein by reference in its entirety.

All literatures, patent applications, and technical standards described in the present specification are incorporated in the present specification by reference to the same extent as in a case where the individual literatures, patent applications, and technical standards are specifically and individually stated to be incorporated by reference.
[Sequence list] International application 21F00801W1JP22035787_11.xml based on International Patent Cooperation Treaty

## Claims

1. A production method of a polypeptide in which expressing a polypeptide as a tagged polypeptide is included, the production method comprising:
expressing the tagged polypeptide from a nucleic acid containing
a base sequence encoding a tag consisting of an amino acid sequence set forth in SEQ ID NO: 1, which is arranged immediately after a start codon, and
a base sequence encoding the polypeptide that is arranged in-frame downstream of the base sequence encoding the tag,
SEQ ID NO: 1: Val-Lys-Lys-(Xaa)n (Xaa)n is a chain of n pieces of any amino acids, where n is an integer of 1 to 8, and the amino acids constituting (Xaa)n may be one type of amino acid or two or more types of amino acids.

2. The production method of a polypeptide according to claim 1,
wherein the tag is a tag consisting of an amino acid sequence set forth in SEQ ID NO: 2,
SEQ ID NO: 2: Val-Lys-Lys-Xaa-(Xaa)m Xaa at a fourth position from an N-terminal is one type of amino acid selected from the group consisting of Ile, Lys, Arg, His, Ser, and Thr, (Xaa)m is a chain of m pieces of any amino acids, where m is an integer of 0 to 7, and the amino acids constituting (Xaa)m may be one type of amino acid or two or more types of amino acids.

3. The production method of a polypeptide according to claim 1,
wherein the tag is a tag consisting of an amino acid sequence set forth in SEQ ID NO: 3,
SEQ ID NO: 3: Val-Lys-Lys-Xaa-(Xaa)k Xaa at a fourth position from an N-terminal is one type of amino acid selected from the group consisting of Ile, Lys, and Thr, (Xaa)k is a chain of k pieces of amino acids selected from the group consisting of Lys, Thr, and Asp, where k is an integer of 0 to 6, and the amino acids constituting (Xaa)k may be one type of amino acid or two or more types of amino acids.

4. The production method of a polypeptide according to any one of claims 1 to 3,
wherein the tagged polypeptide is expressed from the nucleic acid by a cell-free peptide synthesis system or Escherichia coli.

5. The production method of a polypeptide according to claim 1,
wherein the polypeptide contains an unnatural amino acid.

6. A tag consisting of an amino acid sequence set forth in SEQ ID NO: 1,
SEQ ID NO: 1: Val-Lys-Lys-(Xaa)n (Xaa)n is a chain of n pieces of any amino acids, where n is an integer of 1 to 8, and the amino acids constituting (Xaa)n may be one type of amino acid or two or more types of amino acids.

7. A tag consisting of an amino acid sequence set forth in SEQ ID NO: 2,
SEQ ID NO: 2: Val-Lys-Lys-Xaa-(Xaa)m Xaa at a fourth position from an N-terminal is one type of amino acid selected from the group consisting of Ile, Lys, Arg, His, Ser, and Thr, (Xaa)m is a chain of m pieces of any amino acids, where m is an integer of 0 to 7, and the amino acids constituting (Xaa)m may be one type of amino acid or two or more types of amino acids.

8. A tag consisting of an amino acid sequence set forth in SEQ ID NO: 3,
SEQ ID NO: 3: Val-Lys-Lys-Xaa-(Xaa)k Xaa at a fourth position from an N-terminal is one type of amino acid selected from the group consisting of Ile, Lys, and Thr, (Xaa)k is a chain of k pieces of amino acids selected from the group consisting of Lys, Thr, and Asp, where k is an integer of 0 to 6, and the amino acids constituting (Xaa)k may be one type of amino acid or two or more types of amino acids.

9. The tag according to any one of claims 6 to 8,
wherein the tag is used for expressing a polypeptide as a tagged polypeptide by a cell-free peptide synthesis system or Escherichia coli.

10. The tag according to claim 9,
wherein the polypeptide contains an unnatural amino acid.

11. An expression vector comprising:
a base sequence encoding a tag consisting of an amino acid sequence set forth in SEQ ID NO: 1, which is arranged immediately after a start codon; and
a base sequence encoding a polypeptide that is arranged in-frame downstream of the base sequence encoding the tag,
SEQ ID NO: 1: Val-Lys-Lys-(Xaa)n (Xaa)n is a chain of n pieces of any amino acids, where n is an integer of 1 to 8, and the amino acids constituting (Xaa)n may be one type of amino acid or two or more types of amino acids.

12. The expression vector according to claim 11,
wherein the tag is a tag consisting of an amino acid sequence set forth in SEQ ID NO: 2,
SEQ ID NO: 2: Val-Lys-Lys-Xaa-(Xaa)m Xaa at a fourth position from an N-terminal is one type of amino acid selected from the group consisting of Ile, Lys, Arg, His, Ser, and Thr, (Xaa)m is a chain of m pieces of any amino acids, where m is an integer of 0 to 7, and the amino acids constituting (Xaa)m may be one type of amino acid or two or more types of amino acids.

13. The expression vector according to claim 11,
wherein the tag is a tag consisting of an amino acid sequence set forth in SEQ ID NO: 3,
SEQ ID NO: 3: Val-Lys-Lys-Xaa-(Xaa)k Xaa at a fourth position from an N-terminal is one type of amino acid selected from the group consisting of Ile, Lys, and Thr, (Xaa)k is a chain of k pieces of amino acids selected from the group consisting of Lys, Thr, and Asp, where k is an integer of 0 to 6, and the amino acids constituting (Xaa)k may be one type of amino acid or two or more types of amino acids.

14. The expression vector according to any one of claims 11 to 13,
wherein the expression vector is designed to be expressed in a cell-free peptide synthesis system or Escherichia coli.

15. An evaluation method of a polypeptide, comprising:
producing a polypeptide by the production method of a polypeptide according to claim 1; and
evaluating binding property of the polypeptide to a target substance.

16. A production method of a nucleic acid display library comprising:
producing a nucleic acid-polypeptide conjugate by expressing a tagged polypeptide from a nucleic acid containing a base sequence encoding a tag consisting of an amino acid sequence set forth in SEQ ID NO: 1, which is arranged immediately after a start codon, and a base sequence encoding a polypeptide that is arranged in-frame downstream of the base sequence encoding the tag,
SEQ ID NO: 1: Val-Lys-Lys-(Xaa)n (Xaa)n is a chain of n pieces of any amino acids, where n is an integer of 1 to 8, and the amino acids constituting (Xaa)n may be one type of amino acid or two or more types of amino acids.

17. The production method of a nucleic acid display library according to claim 16,
wherein the tag is a tag consisting of an amino acid sequence set forth in SEQ ID NO: 2,
SEQ ID NO: 2: Val-Lys-Lys-Xaa-(Xaa)m Xaa at a fourth position from an N-terminal is one type of amino acid selected from the group consisting of Ile, Lys, Arg, His, Ser, and Thr, (Xaa)m is a chain of m pieces of any amino acids, where m is an integer of 0 to 7, and the amino acids constituting (Xaa)m may be one type of amino acid or two or more types of amino acids.

18. The production method of a nucleic acid display library according to claim 16,
wherein the tag is a tag consisting of an amino acid sequence set forth in SEQ ID NO: 3,
SEQ ID NO: 3: Val-Lys-Lys-Xaa-(Xaa)k Xaa at a fourth position from an N-terminal is one type of amino acid selected from the group consisting of Ile, Lys, and Thr, (Xaa)k is a chain of k pieces of amino acids selected from the group consisting of Lys, Thr, and Asp, where k is an integer of 0 to 6, and the amino acids constituting (Xaa)k may be one type of amino acid or two or more types of amino acids.

19. The production method of a nucleic acid display library according to claim 16,
wherein the production of the nucleic acid-polypeptide conjugate includes producing an mRNA-polypeptide conjugate and reverse-transcribing an mRNA of the mRNA-polypeptide conjugate to produce a cDNA-polypeptide conjugate.

20. The production method of a nucleic acid display library according to claim 16,
wherein in the nucleic acid-polypeptide conjugate, the nucleic acid and the polypeptide are linked by a puromycin linker.

21. The production method of a nucleic acid display library according to claim 16,
wherein the polypeptide contains an unnatural amino acid.

22. The production method of a nucleic acid display library according to claim 16,
wherein the polypeptide is a polypeptide containing an amino acid having a first functional group and an amino acid having a second functional group that is covalently bonded to the first functional group, in which 2 to 28 amino acids are interposed between the amino acid having the first functional group and the amino acid having the second functional group, and
the production method further includes allowing the polypeptide of the nucleic acid-polypeptide conjugate to cyclize by a covalent bond between the first functional group and the second functional group.

23. A screening method comprising:
producing a nucleic acid display library by the production method of a nucleic acid display library according to claim 16; and
selecting a nucleic acid-polypeptide conjugate having a target activity from the nucleic acid display library and identifying a base sequence of a nucleic acid of the selected nucleic acid-polypeptide conjugate.
